(19) Europäisches Patentamt
European Patent Office
Office européen des brevets

(11) EP 0 727 406 B1

(12) **EUROPÄISCHE PATENTSCHRIFT**

(45) Veröffentlichungstag und Bekanntmachung des Hinweises auf die Patenterteilung:
**05.09.2001 Patentblatt 2001/36**

(51) Int Cl.⁷: **C07C 25/18**, C09K 19/30, C07C 43/225, C07C 49/813

(21) Anmeldenummer: **95118849.9**

(22) Anmeldetag: **30.11.1995**

(54) **Vinylenverbindungen und flüssigkristallines Medium**
Vinylene compounds and liquid crystalline medium
Composés du vinylène et milieu cristal liquide

(84) Benannte Vertragsstaaten:
**DE GB**

(30) Priorität: **16.02.1995 DE 19505189**

(43) Veröffentlichungstag der Anmeldung:
**21.08.1996 Patentblatt 1996/34**

(73) Patentinhaber: **MERCK PATENT GmbH**
**64293 Darmstadt (DE)**

(72) Erfinder:
- **Bartmann, Ekkehard, Dr.**
  **D-64390 Erzhausen (DE)**
- **Schoen, Sabine, Dr.**
  **D-64287 Darmstadt (DE)**
- **Tarumi, Kazuaki, Dr.**
  **D-64342 Seeheim-Jugenheim (DE)**

(56) Entgegenhaltungen:
**EP-A- 0 571 916** **EP-A- 0 588 291**
**WO-A-95/30723**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 727 406 B1

Printed by Jouve, 75001 PARIS (FR)

## Beschreibung

Die Erfindung betrifft ein flüssigkristallines Medium auf Basis eines Gemisches von polaren Verbindungen mit positiver dielektrischer Anisotropie, dadurch gekennzeichnet, daß es ein oder mehrere Vinylenverbindungen der Formel I,

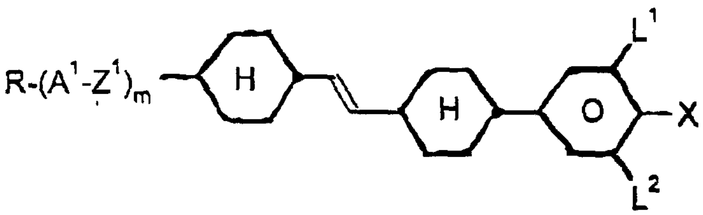

I

worin

- R einen unsubstituierten, einen einfach durch CN oder $CF_3$ oder einen mindestens einfach durch Halogen substituierten Alkenylrest mit 2 bis 15 C-Atomen, wobei in diesen Resten auch eine oder mehrere $CH_2$-Gruppen jeweils unabhängig voneinander durch -O-, -S-, -CO-, -CO-O, -O-CO- oder -O-CO-O- so ersetzt sein können, daß O-Atome nicht direkt miteinander verknüpft sind,

- $A^1$ (a) trans-1,4-Cyclohexylenrest, worin auch eine oder mehrere nicht benachbarte $CH_2$-Gruppen durch -O- und/oder -S- ersetzt sein können,

  (b) 1,4-Phenylenrest, worin auch eine oder zwei CH-Gruppen durch N ersetzt sein können,

  (c) Rest aus der Gruppe 1,4-Cyclohexenylen, 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,

wobei die Reste (a) und (b) ein- oder mehrfach durch CN oder Fluor substituiert sein können,

- $Z^1$ -CO-O-, -O-CO-, $-CH_2O-$, $-OCH_2-$, $-CH_2CH_2-$, -CH=CH-, -C≡C- oder eine Einfachbindung, und

- X CN, OCN, NCS, Cl, F, halogeniertes Alkyl oder Alkenyl mit 1 bis 7 C-Atomen, worin auch eine oder mehrere $CH_2$-Gruppen durch -O- ersetzt sein können,

- $L^1$ und $L^2$ jeweils F, und

- m 0 oder 1

bedeuten,
und zusätzlich ein oder mehrere Verbindungen ausgewählt aus der Gruppe bestehend aus den allgemeinen Formeln II bis VI,

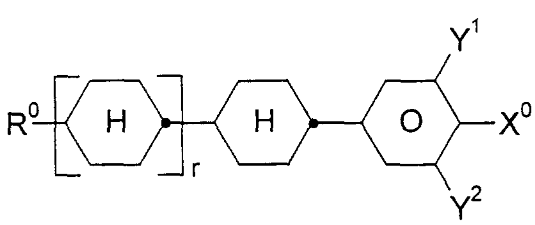

II

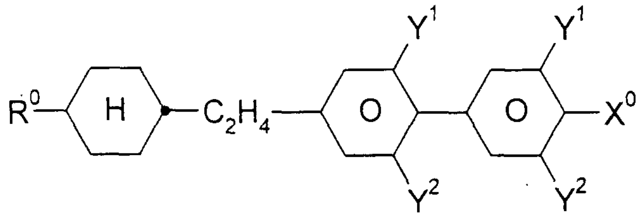

III

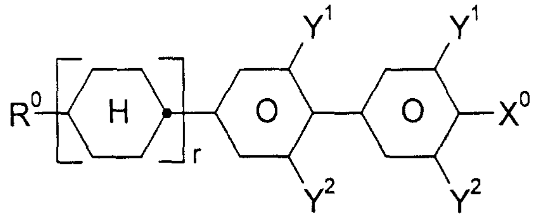

IV

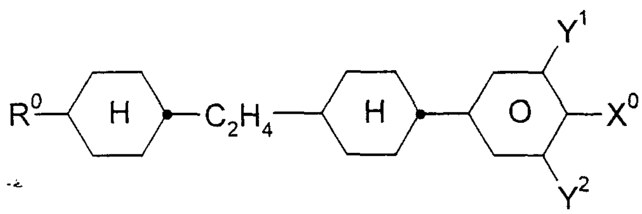

V

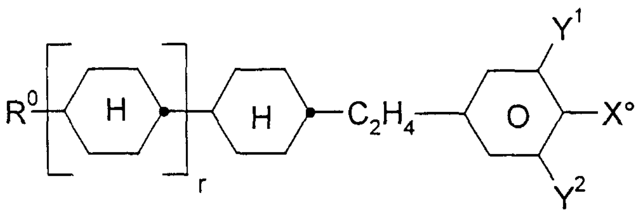

VI

worin

| | |
|---|---|
| $R^0$ | n-Alkyl, Oxaalkyl, Fluoralkyl oder Alkenyl mit jeweils bis zu 9 C-Atomen, |
| $X^0$ | F, Cl, halogeniertes Alkyl, Alkenyl oder Alkoxy mit 1 bis 6 C-Atomen, |
| $Y^1$ und $Y^2$ | jeweils unabhängig voneinander H oder F, und |
| r | 0 oder 1 |

bedeuten,
enthält.

Die Erfindung betrifft weiterhin die Verwendung dieser Verbindungen als Komponenten flüssigkristalliner Medien sowie Flüssigkristall- und elektrooptische Anzeigeelemente, die die erfindungsgemäßen flüssigkristallinen Medien enthalten.

Die Verbindungen der Formel I können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Medien geeignet sind und insbesondere gleichzeitig eine vergleichsweise geringe Viskosität besitzen sowie eine relativ hohe dielektrische Anisotropie.

Ähnliche Verbindungen der Formel

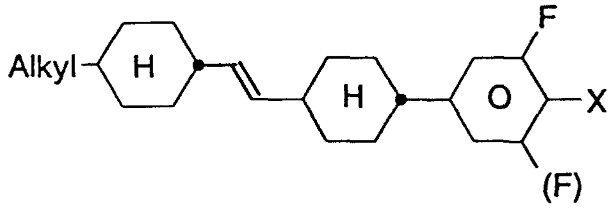

X = F oder $CF_3$ sind bereits aus der US 5,358,662 und WO 94/20443 bekannt.

Es wurde nun gefunden, daß Verbindungen der Formel I als Komponenten flüssigkristalliner Medien vorzüglich geeignet sind. Insbesondere verfügen sie über vergleichsweise niedrige Viskositäten. Mit ihrer Hilfe lassen sich stabile flüssigkristalline Medien mit breitem Mesophasenbereich und vorteilhaften Werten für die optische und dielektrische Anisotropie erhalten. Diese Medien weisen ferner ein sehr gutes Tieftemperaturverhalten auf.

Im Hinblick auf die verschiedensten Einsatzbereiche derartiger Verbindungen mit hohem $\Delta\varepsilon$ war es jedoch wünschenswert, weitere Verbindungen zur Verfügung zu haben, die auf die jeweiligen Anwendungen genau maßgeschneiderte Eigenschaften aufweisen.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind flüssigkristalline Medien mit einem Gehalt an mindestens einer Verbindung der Formel I sowie Flüssigkristallanzeigeelemente, insbesondere elektroopische Anzeigeelemente, die derartige Medien enthalten.

In den Verbindungen der Formel I bedeutet R vorzugsweise einen geradkettigen Alkenylrest, $A^1$ bedeutet bevorzugt 1,4-Phenylen (Phe) oder trans-1,4-Cyclohexylen (Cyc), ferner 1,4-Cyclohexenylen (Che), Pyrimidin-2,5-diyl (Pyr) oder 1,3-Dioxan-2,5-diyl (Dio).

Bevorzugt sind auch Verbindungen der Formel I sowie aller Teilformeln, in denen $A^1$ ein- oder zweifach durch F oder einfach durch CN substituiertes 1,4-Phenylen bedeutet. Insbesondere sind dies 2-Fluor-1,4-phenylen, 3-Fluor-1,4-phenylen und 3,5-Difluor-1,4-phenylen sowie 2-Cyan-1,4-phenylen und 3-Cyan-1,4-phenylen.

$Z^1$ bedeutet bevorzugt eine Einfachbindung, -CO-O-, -O-CO- und $-CH_2CH_2-$, in zweiter Linie bevorzugt $-CH_2O-$ und $-OCH_2-$.

Der Alkenylrest R kann geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3-oder Pent-4-enyl, Hex-1-, 2-, 3-, 4- oder Hex-5-enyl, Hept-1-, 2-, 3-, 4-, 5-oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R einen einfach durch CN oder $CF_3$ substituierten Alkenylrest bedeutet, so ist dieser Rest vorzugsweise geradkettig und die Substitution durch CN oder $CF_3$ in $\bar{\omega}$-Position.

Falls R einen mindestens einfach durch Halogen substituierten Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in $\bar{\omega}$-Position.

Verbindungen der Formel I, die über für Polymerisationsreaktionen geeignete Flügelgruppen R verfügen, eignen sich zur Darstellung flüssigkristalliner Polymerer.

Verbindungen der Formeln I mit verzweigten Flügelgruppen R können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien. Verzweigte Gruppen dieser Art enthalten in der Regel nicht mehr als eine Kettenverzweigung.

Verbindungen der Formel I mit $S_A$-Phasen eignen sich beispielsweise für thermisch adressierte Displays.

Verbindungen der Formel I, die über für Polykondensationen geeignete Flügelgruppen R verfügen, eignen sich zur Darstellung flüssigkristalliner Polykondensate.

Formel I umfaßt sowohl die Racemate dieser Verbindungen als auch die optischen Antipoden sowie deren Gemische.

Unter diesen Verbindungen der Formel I sowie den Unterformeln sind diejenigen bevorzugt, in denen mindestens einer der darin enthaltenden Reste eine der angegebenen bevorzugten Bedeutungen hat.

In den Verbindungen der Formel I sind diejenigen Stereoisomeren bevorzugt, in denen die Ringe Cyc und Piperidin trans-1,4-disubstituiert sind. Diejenigen der vorstehend genannten Formeln, die eine oder mehrere Gruppen Pyridin-2,5-diyl (Pyd), Pyr und/oder Dio enthalten, umschließen jeweils die beiden 2,5-Stellungsisomeren.

Einige ganz besonders bevorzugte kleinere Gruppen von Verbindungen sind diejenigen der Teilformeln I1 bis I3:

[$L^1$, $L^2$: F; R' = H, Ethyl, Methyl oder Propyl; n = 0-3]

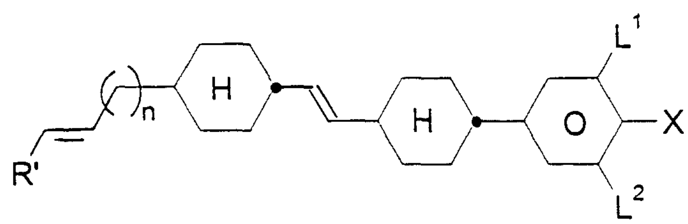

I1

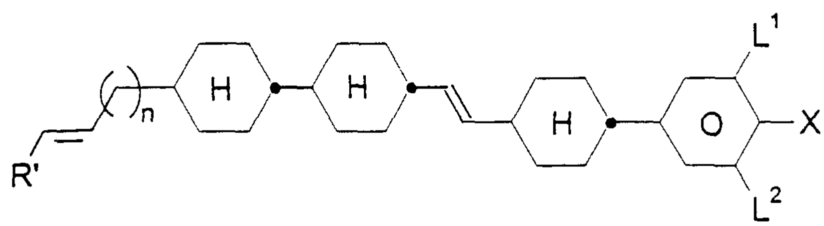

I2

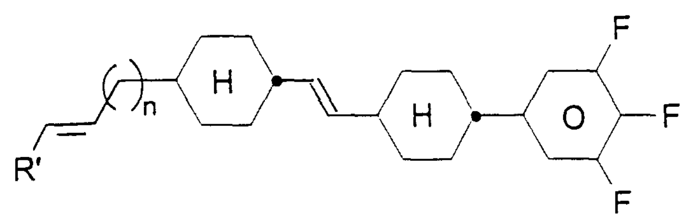

I3

Vorzugsweise ist der Alkenylrest eine $(CH_2)_n$-CH=CH-R'-Gruppe, wobei n = 0-3 und R' H oder Alkyl mit 1-5 C-Atomen, vorzugsweise $CH_3$ und $C_2H_5$, bedeuten.

X ist vorzugsweise F, $OCF_3$, halogeniertes Alkyl, Alkoxy oder Alkenyl mit 1 bis 3 C-Atomen. Halogeniert bedeutet vorzugsweise fluoriert.

X bedeutet insbesondere F, Cl, $CHF_2$, $OCF_3$, $OCHF_2$, $CH=CF_2$, $CF=CF_2$, CF=CHF, CCl=CClF, CH=CHCl, $CH=CH-CF_3$, $OCH=CF_2$, $OCF=CF_2$, OCF=CHF, $OCH=CH-CF_3$, $OCH_2CF_3$, $C_2F_4H$, $C_2F_5$, $OC_2F_4H$, $OC_2F_5$, ferner $(CH_2)_nCF_3$, $O(CH_2)_nCF_3$, $(CH_2)_nCH_2F$, $(CH_2)_nCF_2H$, $O(CH_2)_nCHF_2$, $O(CH_2)_{n-1}CH=CF_2$, $O(CH_2)_{n-1}CF=CF_2$, $C_3F_7$, $OC_3F_7$, wobei n 1 bis 5 bedeutet.

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind.

Dabei kann man auch von an sich bekannten, hier nicht näher erwähnten Varianten Gebrauch machen.

Verbindungen der Formel I können z.B. hergestellt werden, indem man Benzolderivate gemäß folgenden

Reaktionsschemata umsetzt:

Schema 1

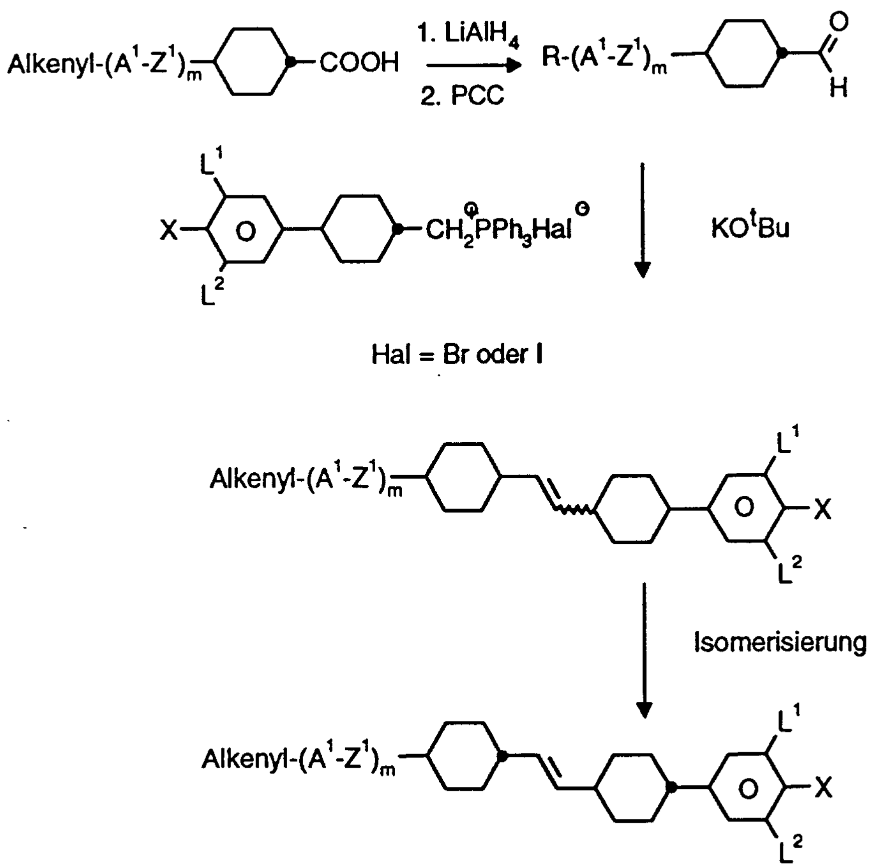

Schema 2

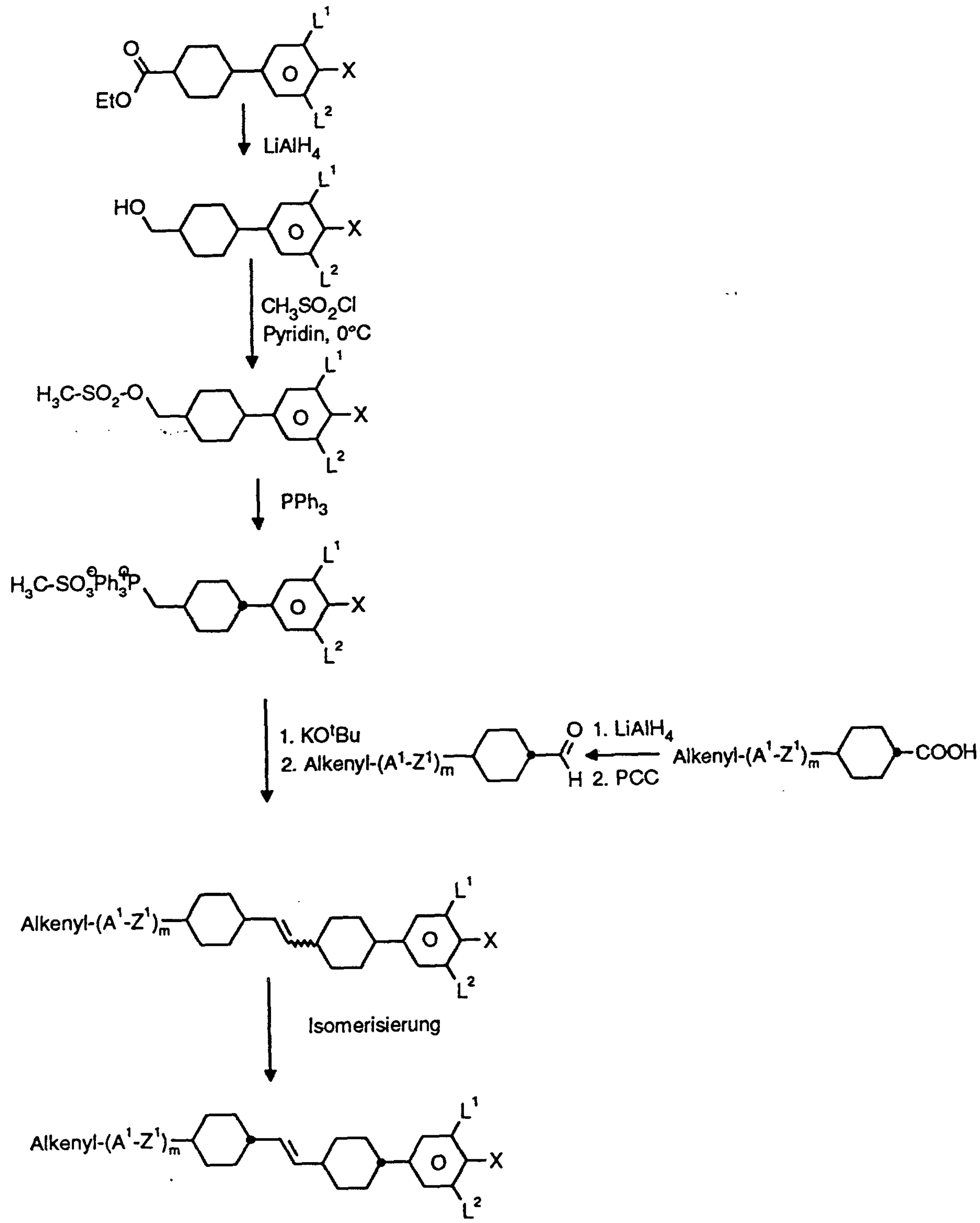
LiAlH4
CH3SO2Cl
Pyridin, 0°C
PPh3
1. KOtBu
2. Alkenyl-(A1-Z1)m
1. LiAlH4
2. PCC
Isomerisierung

Schema 3

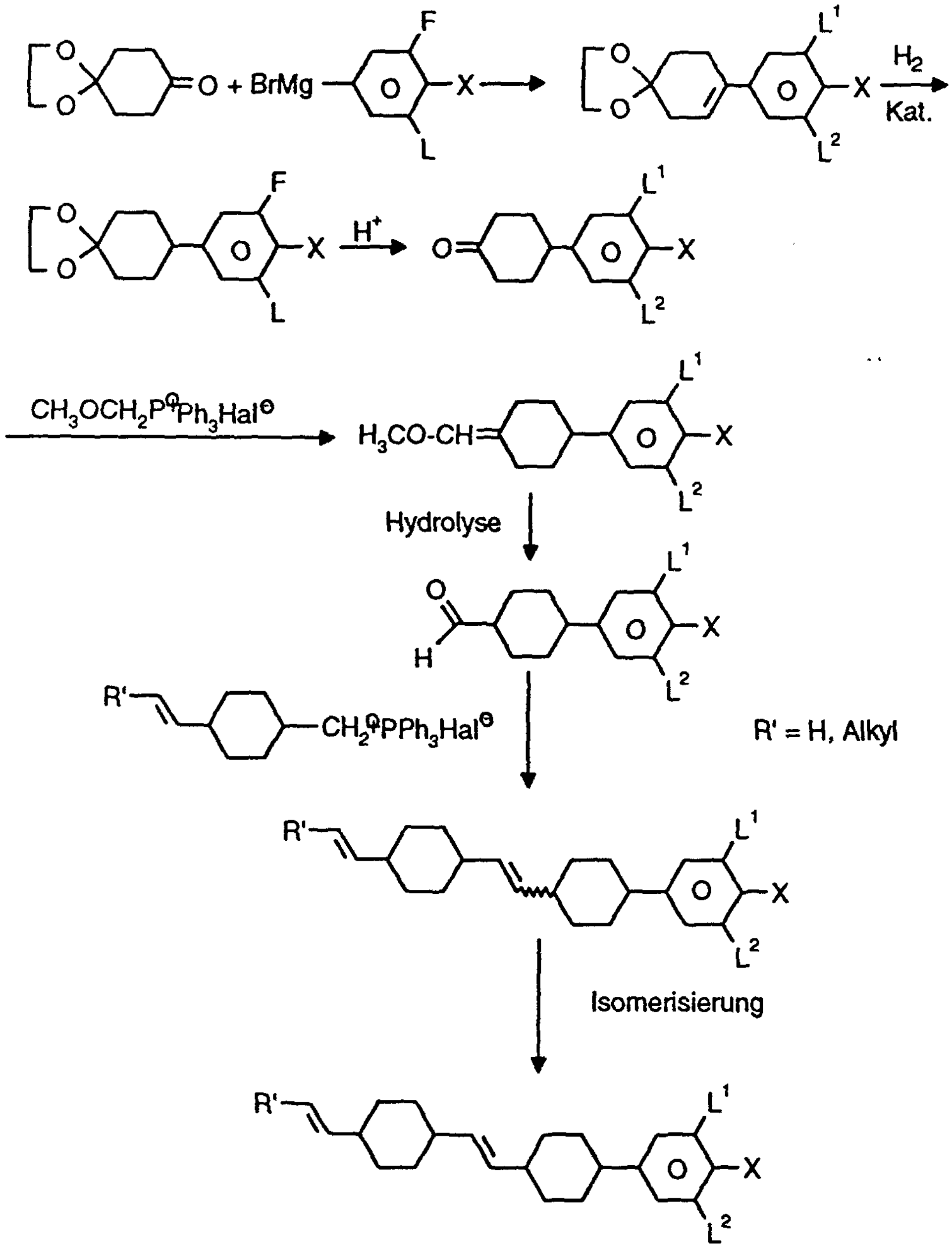
+ BrMg
H2
Kat.
H+
CH3OCH2P⊕Ph3Hal⊖
Hydrolyse
R' = H, Alkyl
Isomerisierung

Schema 4

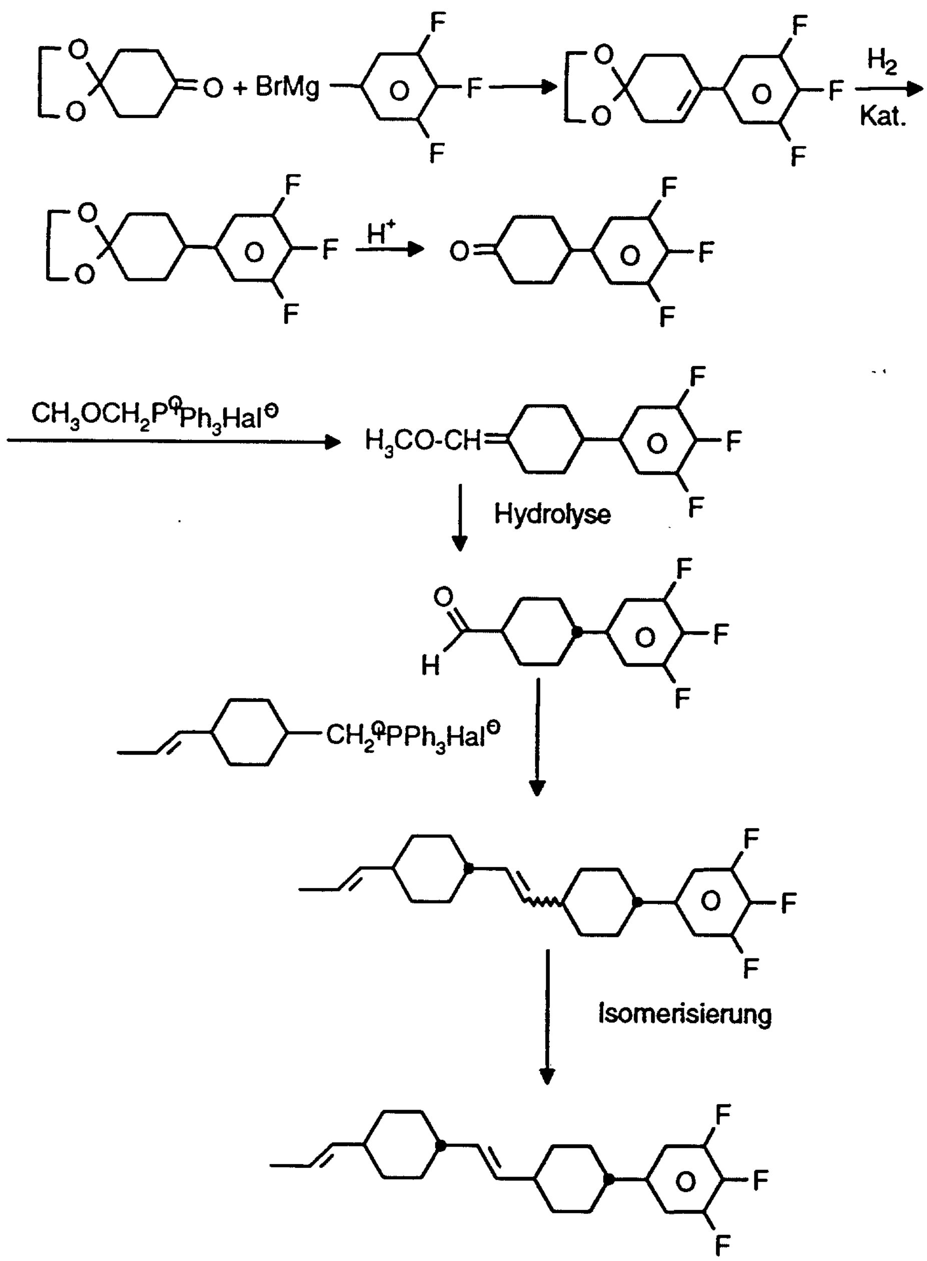


Gegenstand der Erfindung sind auch elektrooptische Anzeigen (insbesondere STN- oder MFK-Anzeigen mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit positiver dielektrischer Anisotropie und hohem spezifischem Widerstand), die derartige Medien enthalten sowie die Verwendung dieser Medien für elektrooptische Zwecke.

Die erfindungsgemäßen Flüssigkristallmischungen ermöglichen eine bedeutende Erweiterung des zur Verfügung stehenden Parameterraumes.

Die erzielbaren Kombinationen aus Klärpunkt, Viskosität bei tiefer Temperatur, thermischer und UV-Stabilität und dielektrischer Anisotropie übertreffen bei weitem bisherige Materialien aus dem Stand der Technik.

Die Forderung nach hohem Klärpunkt, nematischer Phase bei tiefer Temperatur sowie einem hohen $\Delta\varepsilon$ konnte bislang nur unzureichend erfüllt werden. Systeme wie z.B. ZLI-3119 weisen zwar vergleichbaren Klärpunkt und vergleichbar günstige Viskositäten auf, besitzen jedoch ein $\Delta\varepsilon$ von nur +3.

Andere Mischungs-Systeme besitzen vergleichbare Viskositäten und Werte von $\Delta\varepsilon$, weisen jedoch nur Klärpunkte in der Gegend von 60 °C auf.

Die erfindungsgemäßen Flüssigkristallmischungen ermöglichen es bei Beibehaltung der nematischen Phase bis -20 °C und bevorzugt bis -30 °C, besonders bevorzugt bis -40 °C, Klärpunkte oberhalb 80°, vorzugsweise oberhalb 90°, besonders bevorzugt oberhalb 100 °C, gleichzeitig dielektrische Anisotropiewerte $\Delta\varepsilon \geqq 6$, vorzugsweise $\geq 8$ und einen hohen Wert für den spezifischen Widerstand zu erreichen, wodurch hervorragende STN- und MKF-Anzeigen erzielt werden können. Insbesondere sind die Mischungen durch kleine Operationsspannungen gekennzeichnet. Die TN-Schwellen liegen unterhalb 2,0 V, vorzugsweise unterhalb 1,5 V, besonders bevorzugt < 1,3 V.

Es versteht sich, daß durch geeignete Wahl der Komponenten der erfindungsgemäßen Mischungen auch höhere Klärpunkte (z.B. oberhalb 110°) bei höheren Schwellenspannung oder niedrigere Klärpunkte bei niedrigeren Schwellenspannungen unter Erhalt der anderen vorteilhaften Eigenschaften realisiert werden können. Ebenso können bei entsprechend wenig erhöhten Viskositäten Mischungen mit größerem $\Delta\varepsilon$ und somit geringeren Schwellen erhalten werden. Die erfindungsgemäßen MFK-Anzeigen arbeiten vorzugsweise im ersten Transmissionsminimum nach Gooch und Tarry [C.H. Gooch und H.A. Tarry, Electron. Lett. 10, 2-4, 1974; C.H. Gooch und H.A. Tarry, Appl. Phys., Vol. 8, 1575-1584, 1975], wobei hier neben besonders günstigen elektrooptischen Eigenschaften wie z.B. hohe Steilheit der Kennlinie und geringe Winkelabhängigkeit des Kontrastes (DE-PS 30 22 818) bei gleicher Schwellenspannung wie in einer analogen Anzeige im zweiten Minimum eine kleinerere dielektrische Anisotropie ausreichend ist. Hierdurch lassen sich unter Verwendung der erfindungsgemäßen Mischungen im ersten Minimum deutlich höhere spezifische Widerstände verwirklichen als bei Mischungen mit Cyanverbindungen. Der Fachmann kann durch geeignete Wahl der einzelnen Komponenten und deren Gewichtsanteilen mit einfachen Routinemethoden die für eine vorgegebene Schichtdicke der MFK-Anzeige erforderliche Doppelbrechung einstellen.

Die Viskosität bei 20 °C ist vorzugsweise < 60 mPa.s, besonders bevorzugt < 50 mPa.s. Der nematische Phasenbereich ist vorzugsweise mindestens 90°, insbesondere mindestens 100°. Vorzugsweise erstreckt sich dieser Bereich mindestens von -20° bis +80°.

Messungen des "Capacity Holding-ratio" (HR) [S. Matsumoto et al., Liquid Crystals 5, 1320 (1989); K. Niwa et al., Proc. SID Conference, San Francisco, June 1984, p. 304 (1984); G. Weber et al., Liquid Crystals 5, 1381 (1989)] haben ergeben, daß erfindungsgemäße Mischungen enthaltend Verbindungen der Formel I eine deutlich kleinere Abnahme des HR mit steigender Temperatur aufweisen als analoge Mischungen enthaltend anstelle den Verbindungen der Formel I

Cyanophenylcyclohexane der Formel

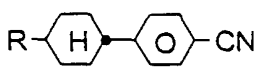

oder Ester der Formel

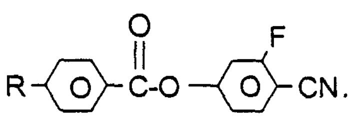

Auch die UV-Stabilität der erfindungsgemäßen Mischungen ist erheblich besser, d. h. sie zeigen eine deutlich kleinere Abnahme des HR unter UV-Belastung.

Vorzugsweise basieren die erfindungsgemäßen Medien auf mehreren (vorzugsweise zwei oder mehr) Verbindungen der Formel I, d.h. der Anteil dieser Verbindungen ist 5-95 %, vorzugsweise 10-60 % und besonders bevorzugt im Bereich von 20-50 %.

Die einzelnen Verbindungen der Formeln II bis VI und deren Unterformeln, die in den erfindungsgemäßen Medien verwendet werden können, sind entweder bekannt, oder sie können analog zu den bekannten Verbindungen hergestellt werden.

Bevorzugte Ausführungsformen sind im folgenden angegeben:

- Die Verbindung der Formel IV ist vorzugsweise

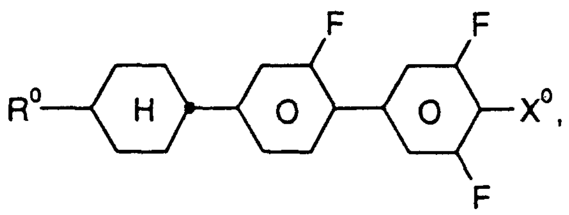

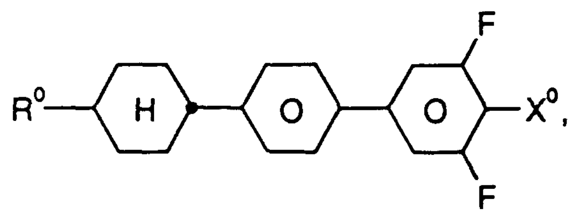

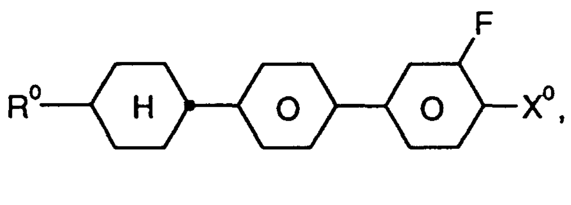

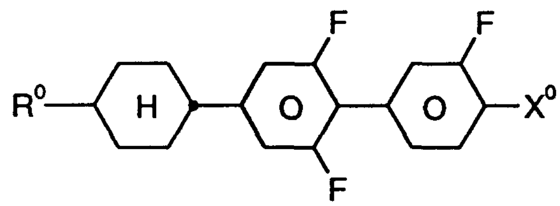

oder

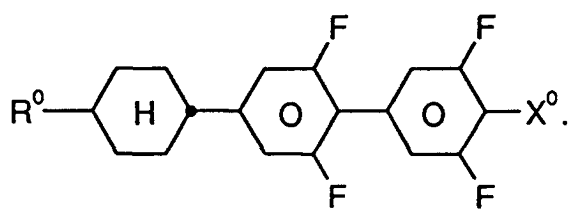

- Medium enthält zusätzlich eine oder mehrere Verbindungen der Formel

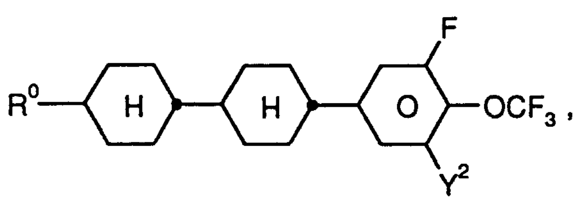

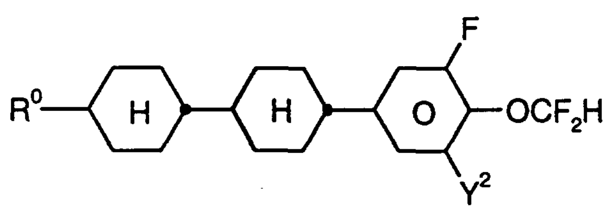

und/oder

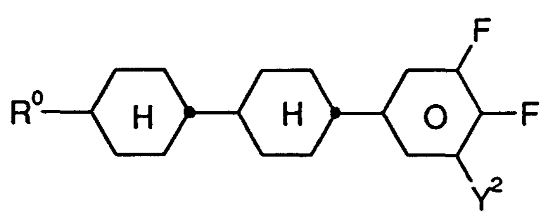

- Medium enthält zusätzlich eine oder mehrere Verbindungen ausgewählt aus der Gruppe bestehend aus den allgemeinen Formeln VII bis XII:

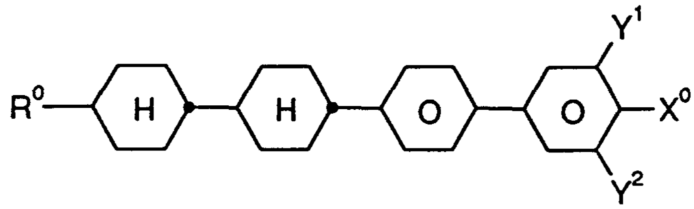

VII

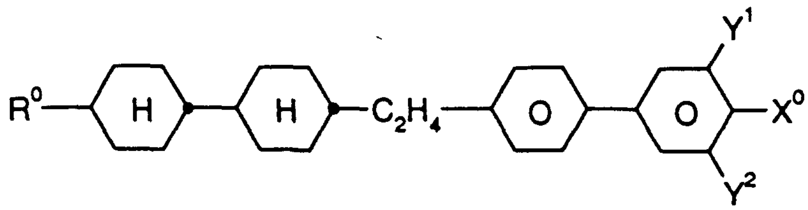

VIII

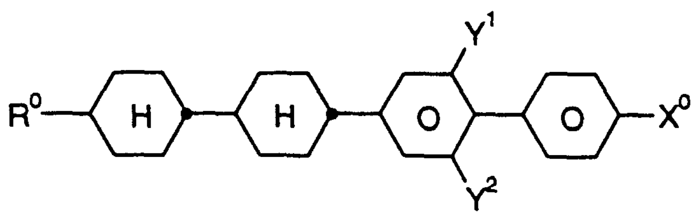

IX

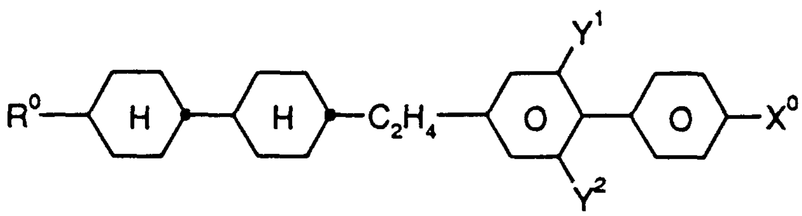

X

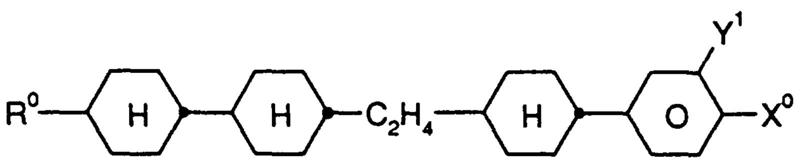

XI

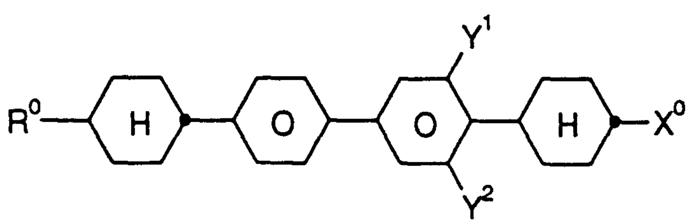

XII

worin $R^0$, $X^0$, $Y^1$ und $Y^2$ jeweils unabhängig voneinander F, Cl, $CF_3$, $OCF_3$, $OCHF_2$, Alkyl, Oxaalkyl, Fluoralkyl

oder Alkenyl mit jeweils bis zu 6 C-Atomen bedeutet.

- Der Anteil an Verbindungen der Formeln I bis VI zusammen beträgt im Gesamtgemisch mindestens 50 Gew.-%;
- der Anteil an Verbindungen der Formel I beträgt im Gesamtgemisch 5 bis 50 Gew.-%;
- der Anteil an Verbindungen der Formeln II bis VI im Gesamtgemisch beträgt 30 bis 70 Gew.-%

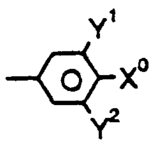 ist vorzugsweise 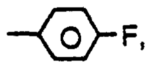, 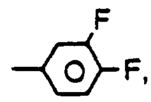, 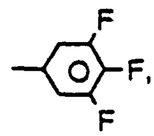,

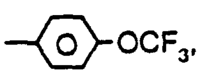, 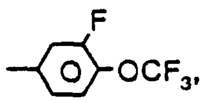, 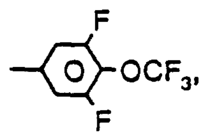, 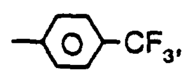,

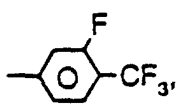, 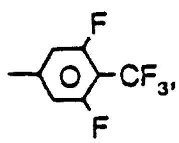, 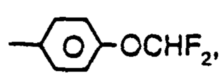, 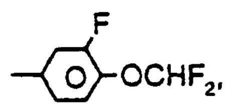,

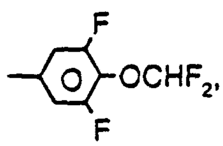, 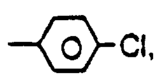, 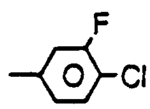 oder 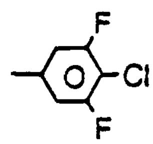

- das Medium enthält Verbindungen der Formeln II, III, IV, V oder VI
- $R^0$ ist geradkettiges Alkyl oder Alkenyl mit 2 bis 7 C-Atomen
- das Medium besteht im wesentlichen aus Verbindungen der Formeln I bis VI
- Das Gewichtsverhältnis I: (II + III + IV + V + VI) ist vorzugsweise 1 : 10 bis 10 : 1.
- Medium besteht im wesentlichen aus Verbindungen ausgewählt aus der Gruppe bestehend aus den allgemeinen Formeln I bis XII.

Es wurde gefunden, daß bereits ein relativ geringer Anteil an Verbindungen der Formel I im Gemisch mit einer oder mehreren Verbindungen der Formel II, III, IV, V und/oder VI zu einer beträchtlichen Erniedrigung der Schwellenspannung und zu niedrigen Werten für die Doppelbrechung führt, wobei gleichzeitig breite nematische Phasen mit tiefen Übergangstemperaturen smektisch-nematisch beobachtet werden, wodurch die Lagerstabilität verbessert wird. Die Verbindungen der Formeln I bis VI sind farblos, stabil und untereinander und mit anderen Flüssigkristallmaterialien gut mischbar.

Der Ausdruck "Alkyl" umfaßt insbesondere die geradkettigen Gruppen Methyl, Ethyl, Propyl, Butyl, Pentyl, und Hexyl. Gruppen mit 2-5 Kohlenstoffatomen sind im allgemeinen bevorzugt.

Der Ausdruck "Alkenyl" umfaßt insbesondere die geradkettigen Gruppen. Beispiele bevorzugter Alkenylgruppen sind Vinyl, 1 E-Propenyl, 1E-Butenyl, 1E-Pentenyl, 1E-Hexenyl, 3-Butenyl, 3E-Pentenyl, 3E-Hexenyl, 4-Pentenyl, 4Z-Hexenyl, 4E-Hexenyl, 5-Hexenyl, und dergleichen. Gruppen mit bis zu 5 Kohlenstoffatomen sind im allgemeinen bevorzugt.

Der Ausdruck "Fluoralkyl" umfaßt vorzugsweise geradkettige Gruppen mit endständigen Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des

Fluors sind jedoch nicht ausgeschlossen.

Der Ausdruck "Oxaalkyl" umfaßt vorzugsweise geradkettige Reste der Formel $C_nH_{2n+1}$-O-$(CH_2)_m$, worin n und m jeweils unabhängig voneinander 1 bis 6 bedeuten. Vorzugsweise ist n = 1 und m 1 bis 6.

Durch geeignete Wahl der Bedeutungen von $R^0$ und $X^0$ können die Ansprechzeiten, die Schwellenspannung, die Steilheit der Transmissionskennlinien etc. in gewünschter Weise modifiziert werden. Beispielsweise führen 1 E-Alkenylreste, 3E-Alkenylreste, 2E-Alkenyloxyreste und dergleichen in der Regel zu kürzeren Ansprechzeiten, verbesserten nematischen Tendenzen und einem höheren Verhältnis der elastischen Konstanten $k_{33}$ (bend) und $k_{11}$ (splay) im Vergleich zu Alkyl- bzw. Alkoxyresten. 4-Alkenylreste, 3-Alkenylreste und dergleichen ergeben im allgemeinen tiefere Schwellenspannungen und kleinere Werte von $k_{33}/k_{11}$ im Vergleich zu Alkyl- und Alkoxyresten.

Eine $-CH_2CH_2-$-Gruppe führt im allgemeinen zu höheren Werten von $k_{33}/k_{11}$ im Vergleich zu einer einfachen Kovalenzbindung. Höhere Werte von $k_{33}/k_{11}$ ermöglichen z.B. flachere Transmissionskennlinien in TN-Zellen mit 90° Verdrillung (zur Erzielung von Grautönen) und steilere Transmissionskennlinien in STN-, SBE- und OMI-Zellen (höhere Multiplexierbarkeit) und umgekehrt.

Das optimale Mengenverhältnis der Verbindungen der Formeln I und II + III + IV + V + VI hängt weitgehend von den gewünschten Eigenschaften, von der Wahl der Komponenten der Formeln I, II, III, IV, V und/oder VI und von der Wahl weiterer gegebenenfalls vorhandener Komponenten ab. Geeignete Mengenverhältnisse innerhalb des oben angegebenen Bereichs können von Fall zu Fall leicht ermittelt werden.

Die Gesamtmenge an Verbindungen der Formeln I bis XII in den erfindungsgemäßen Gemischen ist nicht kritisch. Die Gemische können daher eine oder mehrere weitere Komponenten enthalten zwecks Optimierung verschiedener Eigenschaften. Der beobachtete Effekt auf die Ansprechzeiten und die Schwellenspannung ist jedoch in der Regel umso größer je höher die Gesamtkonzentration an Verbindungen der Formeln I bis XII ist.

In einer besonders bevorzugten Ausführungsform enthalten die erfindungsgemäßen Medien Verbindungen der Formel II bis VI (vorzugsweise II und/oder III), worin $X^0$ $OCF_3$, $OCHF_2$, F, $OCH=CF_2$, $OCF=CF_2$ oder $OCF_2$-$CF_2H$ bedeutet. Eine günstige synergistische Wirkung mit den Verbindungen der Formel I führt zu besonders vorteilhaften Eigenschaften.

Gegenstand der Erfindung ist ein flüssigkristallines Medium auf Basis eines Gemisches von polaren Verbindungen mit positiver dielektrischer Anisotropie, dadurch gekennzeichnet, daß es ein oder mehrere Verbindungen der Formel I und zusätzlich ein oder mehrere Verbindungen der Formeln II bis VI, ferner ein oder mehrere Verbindungen der Formeln VII bis XII enthält. Derartige Medien sind insbesondere geeignet für MFK-Anzeigen.

Die erfindungsgemäßen Medien können ferner eine Komponente A enthalten bestehend aus einer oder mehreren Verbindungen mit einer dielektrischen Anisotropie von -1,5 bis +1,5 der allgemeinen Formel I'

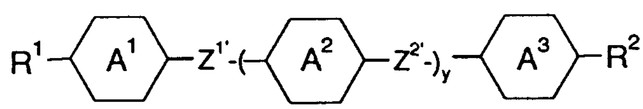

I'

worin

| | |
|---|---|
| $R^1$ und $R^2$ | jeweils unabhängig voneinander n-Alkyl, ω-Fluoralkyl oder n-Alkenyl mit bis zu 9 C-Atomen, |
| die Ringe | $A^1$, $A^2$ und $A^3$<br>jeweils unabhängig voneinander 1,4-Phenylen, 2- oder 3-Fluor-1,4-phenylen, trans-1,4-Cyclohexylen oder 1,4-Cyclohexenylen, |
| $Z^{1'}$ und $Z^{2'}$ | jeweils unabhängig voneinander $-CH_2CH_2-$, -C≡C-, -CO-O-, -O-CO- oder eine Einfachbindung, |

und

y 0, 1 oder 2 bedeutet.

Komponente A enthält vorzugsweise eine oder mehrere Verbindungen ausgewählt aus der Gruppe bestehend aus II1 bis II7:

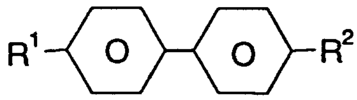

II1

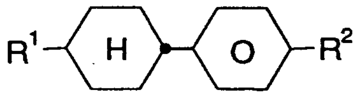

II2

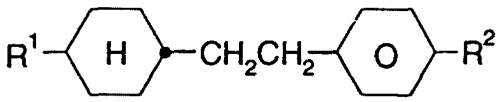

II3

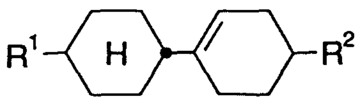

II4

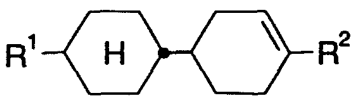

II5

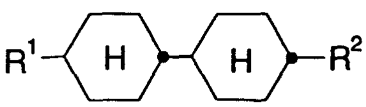

II6

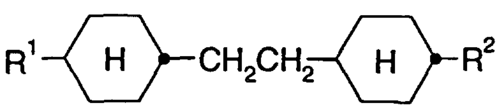

II7

worin $R^1$ und $R^2$ die bei Formel I' angegebene Bedeutung haben.

Vorzugsweise enthält Komponente A zusätzlich eine oder mehrere Verbindungen ausgewählt aus der Gruppe bestehend aus 118 bis II20:

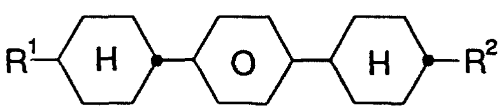

II8

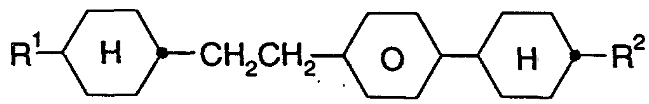

II9

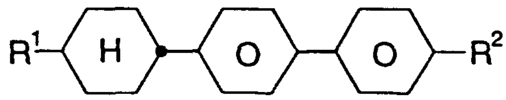

II10

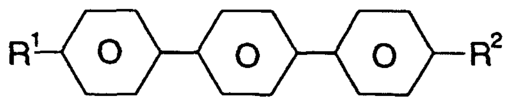

II11

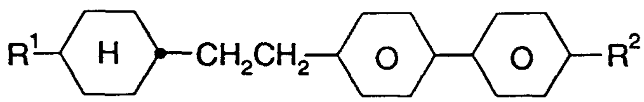

II12

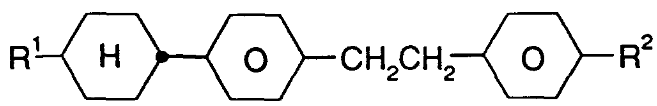

II13

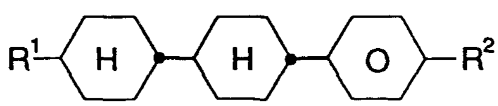

II14

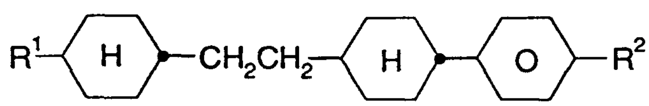

II15

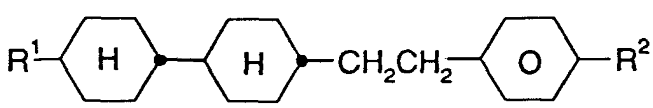

II16

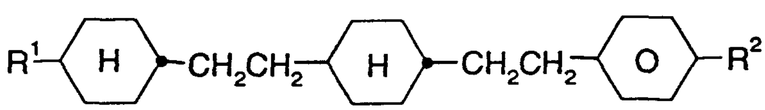

II17

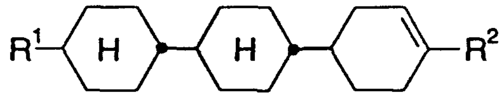

II18

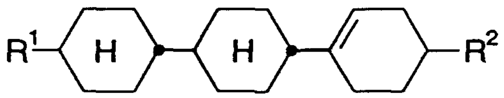

II19

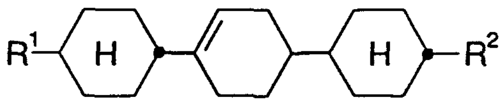

II20

worin $R^1$ und $R^2$ die bei Formel I'angegebene Bedeutung haben und die 1,4-Phenylengruppen in II8 bis II17 jeweils unabhängig voneinander auch durch Fluor ein- oder mehrfach substituiert sein können.

Ferner enthält Komponente A vorzugsweise zusätzlich eine oder mehrere Verbindungen ausgewählt aus der Gruppe bestehend aus II21 bis II25:

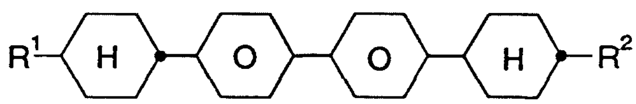

II21

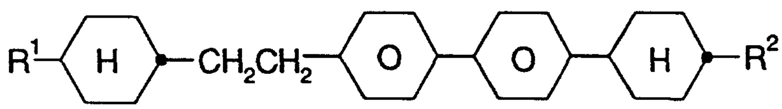

II22

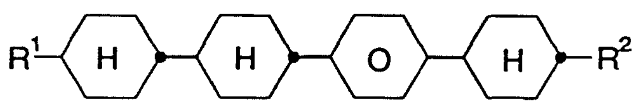

II23

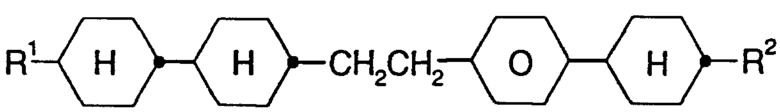

II24

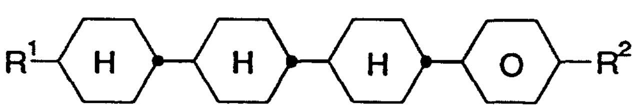

II25

worin $R^1$ und $R^2$ die bei Formel I'angegebene Bedeutung haben und die 1,4-Phenylengruppen in II21 bis II125 jeweils

unabhängig voneinander auch durch Fluor ein- oder mehrfach substituiert sein können.

Schließlich sind derartige Mischungen bevorzugt, deren Komponente A eine oder mehrere Verbindungen ausgewählt aus der Gruppe bestehend aus II26 und II27 enthält:

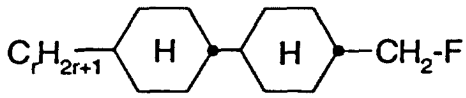

II26

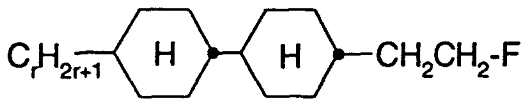

II27

worin $C_rH_{2r+1}$ eine geradkettige Alkylgruppe mit bis zu 7 C-Atomen ist.

In einigen Fällen erweist sich der Zusatz von Verbindungen der Formel

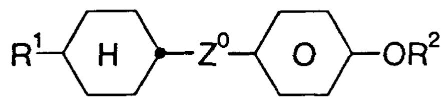

worin
$R^1$ und $R^2$ die bei Formel I' angegebene Bedeutung haben
und

$Z^0$ eine Einfachbindung, $-CH_2CH_2-$,

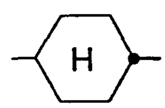

oder

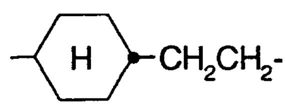

bedeutet,
zur Unterdrückung smektischer Phasen als vorteilhaft, obwohl hierdurch der spezifische Widerstand erniedrigt werden kann. Zur Erzielung von für die Anwendung optimaler Parameterkombinationen kann der Fachmann leicht feststellen, ob und falls ja in welcher Menge diese Verbindungen zugesetzt sein können. Normalerweise werden weniger als 15 %, insbesondere 5-10 % verwendet.

Ferner bevorzugt sind Flüssigkristallmischungen, die eine oder mehrere Verbindungen ausgewählt aus der Gruppe bestehend aus III' und IV' enthalten:

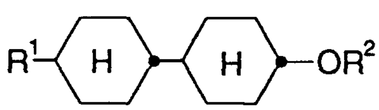

III'

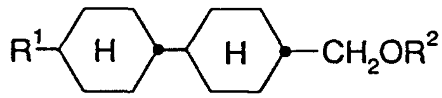

IV'

worin $R^1$ und $R^2$ die bei Formel I'angegebene Bedeutung haben.

Die Art und Menge der polaren Verbindungen mit positiver dielektrischer Anisotropie ist an sich nicht kritisch. Der Fachmann kann unter einer großen Palette bekannter und in vielen Fällen auch kommerziell verfügbarer Komponenten und Basisgemische in einfachen Routineversuchen geeignete Materialien auswählen. Vorzugsweise enthalten die erfindungsgemäßen Medien eine oder mehrere Verbindungen der Formel "

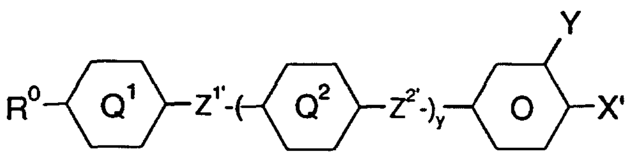

I"

worin $Z^{1'}$, $Z^{2'}$ und y die bei Formel I' angegebene Bedeutung haben, $Q^1$ und $Q^2$ jeweils unabhängig voneinander 1,4-Phenylen, trans-1,4-Cyclohexylen oder 3-Fluor-1,4-phenylen- oder einer der Reste $Q^1$ und $Q^2$ auch trans-1,3-Dioxan-2,5-diyl, Pyrimidin-2,5-diyl, Pyridin-2,5-diyl oder 1,4-Cyclohexenylen bedeutet, $R^0$ n-Alkyl, n-Alkenyl, n-Alkoxy oder n-Oxaalkyl mit jeweils bis zu 9 C-Atome, Y H oder F und X' CN, Halogen, $CF_3$, $OCF_3$ oder $OCHF_2$ ist.

In einer weiteren Ausführungsform basieren die erfindungsgemäßen Medien für STN- oder TN-Anwendungen auf Verbindungen der Formel I" worin X' CN bedeutet. Es versteht sich, daß auch kleinere oder größere Anteile von anderen Verbindungen der Formel I" (X' # CN) in Frage kommen. Für MFK-Anwendungen enthalten die erfindungsgemäßen Medien vorzugsweise nur bis zu ca. 10 % an Nitrilen der Formel I" (vorzugsweise jedoch keine Nitrile der Formel I", sondern Verbindungen der Formel I' mit X' = Halogen, $CF_3$, $OCF_3$ oder $OCHF_2$). Diese Medien basieren vorzugsweise auf den Verbindungen der Formeln II bis XII.

Der Aufbau der erfindungsgemäßen MFK- bzw. STN-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefaßt und umfaßt auch alle Abwandlungen und Modifikationen der MFK-Anzeige, insbesondere auch Matrix-Anzeigeelemente auf Basis poly-Si TFT oder MIM.

Ein wesentlicher Unterschied der erfindungsgemäßen Anzeigen zu den bisher üblichen auf der Basis der verdrillten nematischen Zelle besteht jedoch in der Wahl der Flüssigkristallparameter der Flüssigkristallschicht.

Die Herstellung der erfindungsgemäß verwendbaren Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich, Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation.

Die Dielektrika können auch weitere, dem Fachmann bekannte und in der Literatur beschriebene Zusätze enthalten. Beispielsweise können 0-15 % pleochroitische Farbstoffe oder chirale Dotierstoffe zugesetzt werden.

C bedeutet eine kristalline, S eine smektische, $S_c$ eine smektisch C, N eine nematische und I die isotrope Phase.

$V_{10}$ bezeichnet die Spannung für 10 % Transmission (Blickrichtung senkrecht zur Plattenoberfläche). $t_{on}$ bezeichnet die Einschaltzeit und $t_{off}$ die Ausschaltzeit bei einer Betriebsspannung entsprechend dem 2,5fachen Wert von $V_{10}$. $\Delta n$ bezeichnet die optische Anisotropie und $n_o$ den Brechungsindex. $\Delta\varepsilon$ bezeichnet die dielektrische Anisotropie ($\Delta\varepsilon = \varepsilon_{\parallel} - \varepsilon_{\perp}$, wobei $\varepsilon_{\parallel}$ die Dielektrizitätskonstante parallel zu den Moleküllängsachsen und $\varepsilon_{\perp}$ die Dielektrizitätskonstante senkrecht dazu bedeutet). Die elektrooptischen Daten wurden in einer TN-Zelle im 1. Minimum (d.h. bei einem $d \cdot \Delta n$-Wert von 0,5) bei 20 °C gemessen, sofern nicht ausdrücklich etwas anderes angegeben wird. Die optischen Daten wurden bei 20 °C gemessen, sofern nicht ausdrücklich etwas anderes angegeben wird.

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste $C_nH_{2n+1}$ und $C_mH_{2m+1}$ sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten $R^1$, $R^2$, $L^1$ und $L^2$:

| Code für $R^1$, $R^2$, $L^1$, $L^2$ | $R^1$ | $R^2$ | $L^1$ | $L^2$ |
|---|---|---|---|---|
| nm | $C_nH_{2n+1}$ | $C_mH_{2m+1}$ | H | H |
| nOm | $C_nH_{2n+1}$ | $OC_mH_{2m+1}$ | H | H |
| nO.m | $OC_nH_{2n+1}$ | $C_mH_{2m+1}$ | H | H |
| n | $C_nH_{2n+1}$ | CN | H | H |
| nN.F | $C_nH_{2n+1}$ | CN | F | H |
| nF | $C_nH_{2n+1}$ | F | H | H |
| nOF | $OC_nH_{2n+1}$ | F | H | H |
| nCl | $C_nH_{2n+1}$ | Cl | H | H |
| nF.F | $C_nH_{2n+1}$ | F | F | H |
| nF.F.F | $C_nH_{2n+1}$ | F | F | F |
| $nCF_3$ | $C_nH_{2n+1}$ | $CF_3$ | H | H |
| $nOCF_3$ | $C_nH_{2n+1}$ | $OCF_3$ | H | H |
| $nOCF_2$ | $C_nH_{2n+1}$ | $OCHF_2$ | H | H |
| nS | $C_nH_{2n+1}$ | NCS | H | H |
| rVsN | $C_rH_{2r+1}$-CH=CH-$C_sH_{2s}$- | CN | H | H |
| rEsN | $C_rH_{2r+1}$-O-$C_2H_{2s}$- | CN | H | H |
| nAm | $C_nH_{2n+1}$ | $COOC_mH_{2m+1}$ | H | H |

Die in den Tabellen A und B aufgeführten Verbindungen sind bevorzugte Komponenten in den erfindungsgemäßen Mischungen.

Tabelle A:

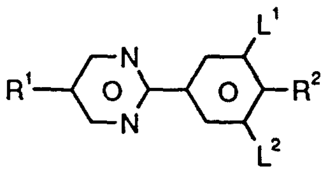

PYP

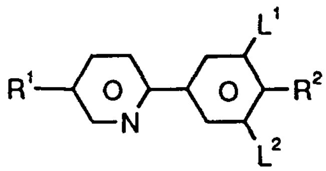

PYRP

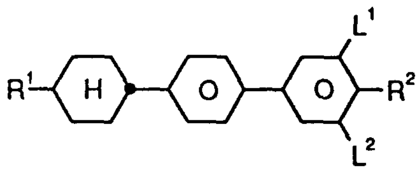

BCH

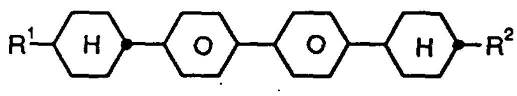

CBC

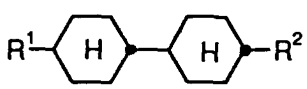

CCH

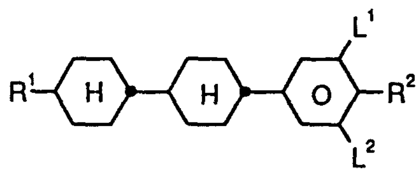

CCP

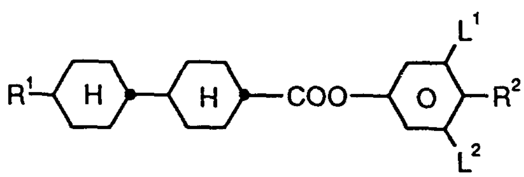

CP

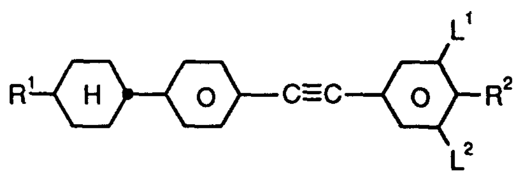

CPTP

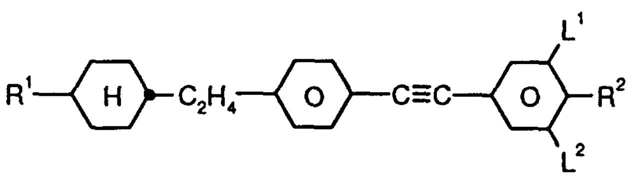

CEPTP

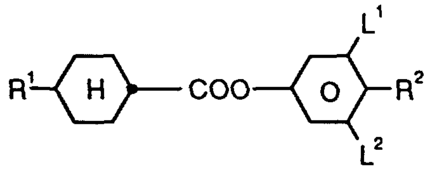

D

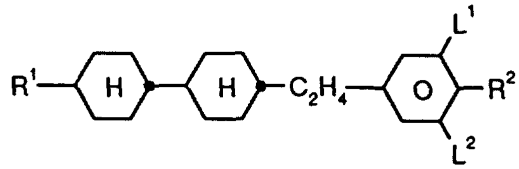

ECCP

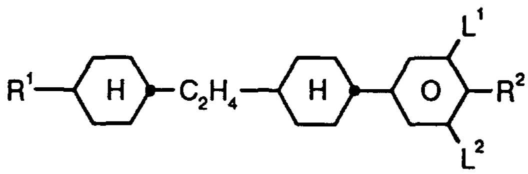

CECP

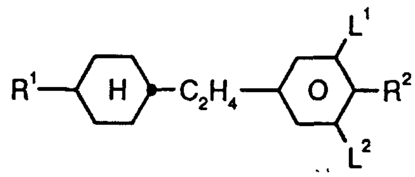

EPCH

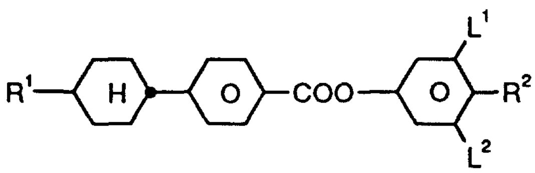

HP

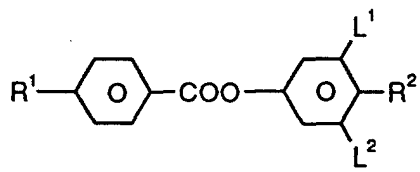

ME

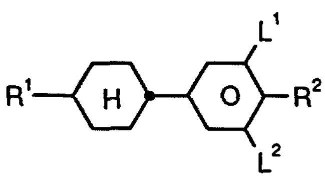

PCH

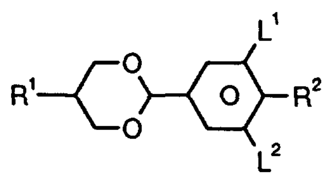

PDX

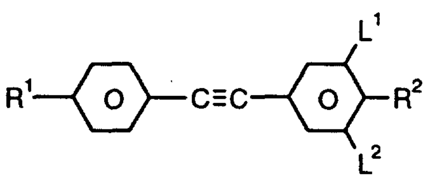

PTP

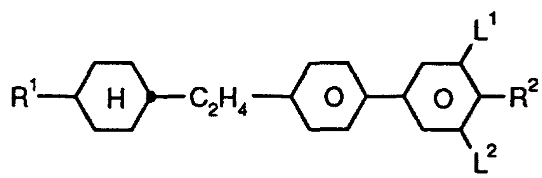

BECH

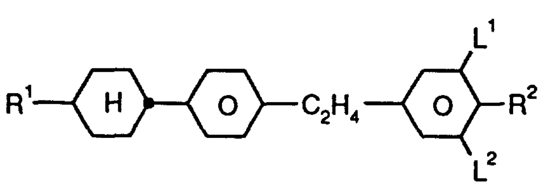

EBCH

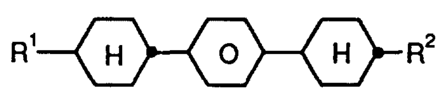

CPC

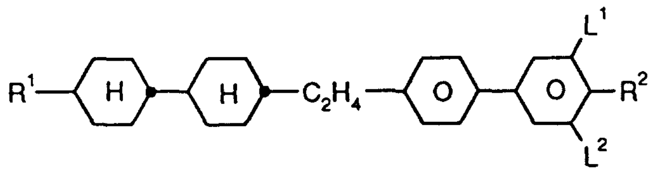

**CCEB**

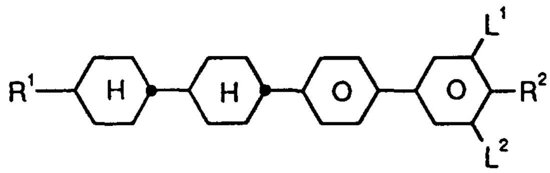

**CCB**

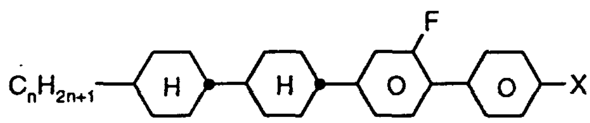

**CCB-n.FX**

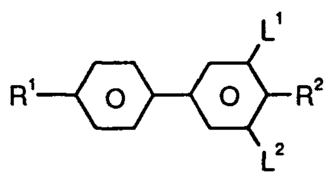

**B**

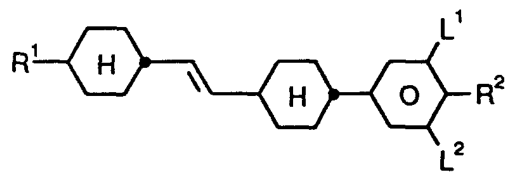

**CVCP**

**Tabelle B:**

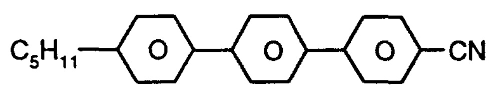

**T15**

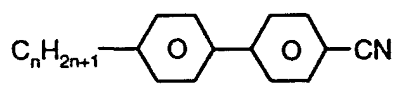

**K3n**

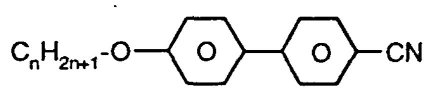

**M3n**

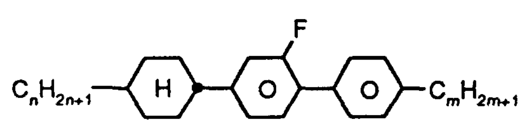

**BCH-n.Fm**

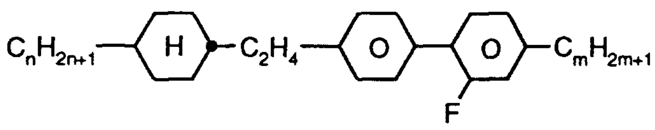

**Inm**

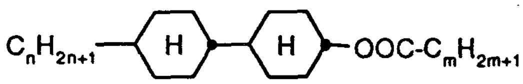

**C-nm**

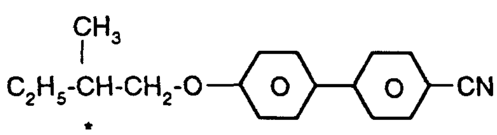

**C15**

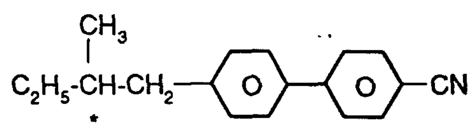

**CB15**

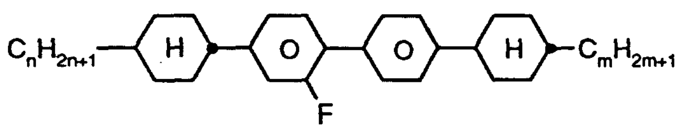

**CBC-nmF**

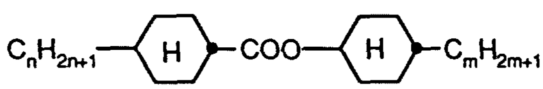

**OS-nm**

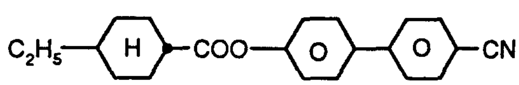

**CHE**

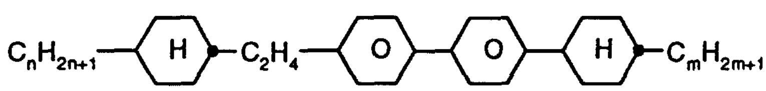

**ECBC-nm**

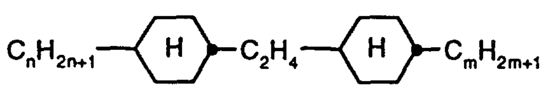

**ECCH-nm**

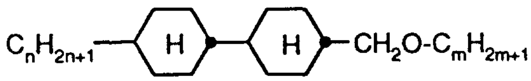

CCH-n1EM

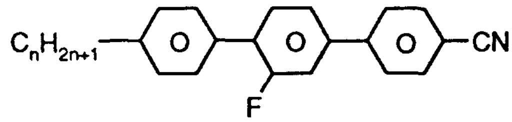

T-nFN

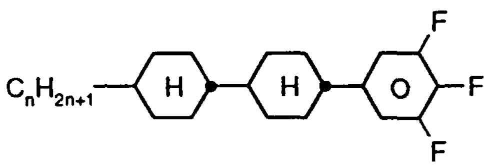

CCP-nF.F.F

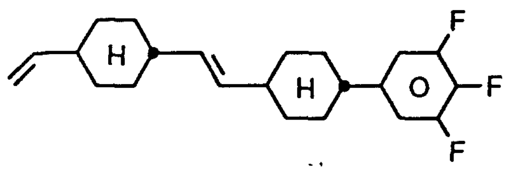

CVCU-V-F

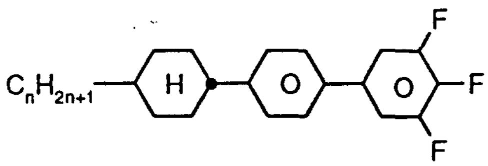

BCH-nF.F.F

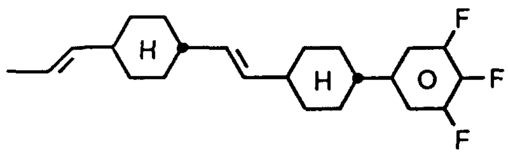

CVCU-1V-F

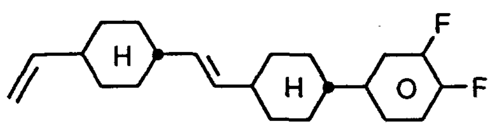

CVCG-V-F

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. $\triangle$n bedeutet optische Anisotropie (589 nm, 20 °C) und die Viskosität ($mm^2$/sec) wurde bei 20 °C bestimmt.

"Übliche Aufarbeitung" bedeutet: man gibt gegebenenfalls Wasser hinzu, extrahiert mit Dichlormethan, Diethylether, Methyl-tert.Butylether, oder Toluol, trennt ab, trocknet die organische Phase, dampft ein und reinigt das Produkt durch Destillation unter reduziertem Druck oder Kristallisation und/oder Chromatographie. Folgende Abkürzungen werden verwendet:

| | |
|---|---|
| BuLi | Butyllithium |
| DAST | Diethylaminoschwefeltrifluorid |
| DCC | Dicyclohexylcarbodiimid |
| DDQ | Dichlordicyanobenzochinon |
| DIBALH | Diisobutylaluminiumhydrid |
| KOT | Kalium-tertiär-butanolat |
| THF | Tetrahydrofuran |
| pTsOH | p-Toluolsulfonsäure |
| TMEDA | Tetramethylethylendiamin |

Beispiel 1

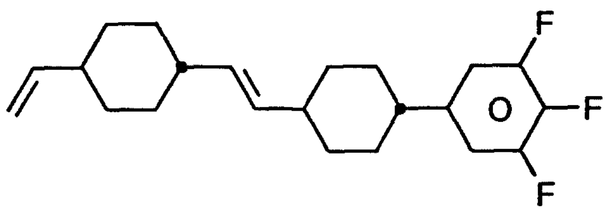

Schritt 1.1

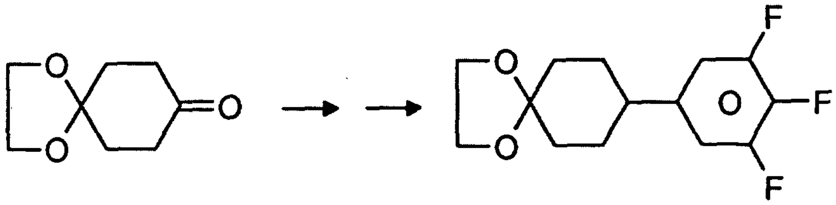

A

0,15 mol Magnesium werden in 50 ml THF in einer Stickstoffatmosphäre vorgelegt und mit 0,15 mol 3,4,5-Trifluorbrombenzol in 100 ml THF versetzt. Man rührt eine Stunde bei Raumtemperatur und tropft eine Lösung bestehend aus 0,15 mol 1,4-Cyclohexandionethylenketal in 100 ml THF zu der Reaktionslösung. Nach 1 h Rühren bei Raumtemperatur wird mit einer gesättigten Ammoniumchloridlösung versetzt. Zuletzt wird wie üblich aufgearbeitet.

Das Reaktionsprodukt wird am Wasserabscheider in der Siedehitze mit p-Toluolsulfonsäure in Toluol dehydriert. Anschließend wird das Reaktionsprodukt am Pd/C-Katalysator hydriert.

Schritt 1.2

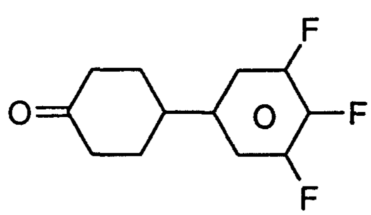

B

Das Produkt aus Schritt 1.1 wird in THF gelöst und in Gegenwart von Ameisensäure 2 h am Rückfluß gekocht. Nach Zugabe von Wasser wird wie üblich aufgearbeitet.

Schritt 1.3

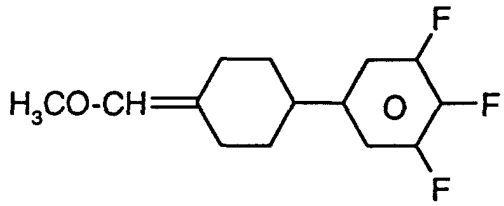

C

In einer Stickstoffatmosphäre werden 2,93 mol Methoxymethyltrimethyltriphenylphosphoniumchlorid und 2,93 mol 4-(3,4,5-Trifluorphenyl)-cyclohexanon aus Schritt 1.2 in 4 l THF gelöst und bei Raumtemperatur unter Rühren mit 2,93 mol Kaliumtert.butylat versetzt. Man rührt 2 h, versetzt mit Wasser und verd. Salzsäure und arbeitet wie üblich auf.

Schritt 1.4

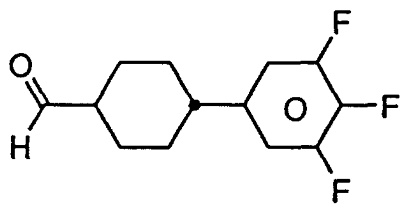

D

50,4 mmol C, 1,0 g 10%ige HCl, 20 ml THF und 50,4 mmol Acetaldehyd werden 0,75 h bei Raumtemperatur gerührt. Man versetzt mit Wasser und Methyl-tert.butylether und arbeitet wie üblich auf.

Schritt 1.5

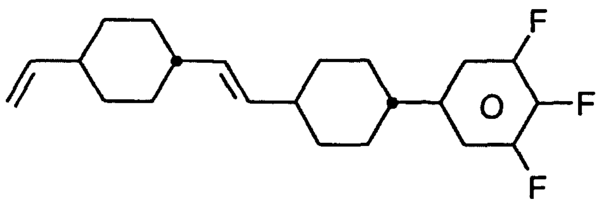

E

0,04 mol D, 0,04 mol 4-(trans-4-Ethenylcyclohexyl)methyl-triphenylphosphoniumiodid und 1140 ml THF werden vorgelegt und portionsweise mit 0,4 mol Kalium-tert.butylat versetzt, wobei die Reaktionstemperatur 35 °C nicht übersteigen sollte. Man rührt über Nacht bei Raumtemperatur, versetzt mit verd. HCl und arbeitet wie üblich auf. Das Rohprodukt wird in Xylol gelöst, mit Iod versetzt und 15 h am Rückfluß gekocht. Man läßt das Reaktionsgemisch abkühlen, versetzt mit einer wäßrigen Natriumhydrogensulfit-Lösung, wäscht mit Wasser und engt im Vakuum ein. Der Rückstand wird aus Ethanol umkristallisiert.
K 89 N (87,1) I; $\Delta n = 0{,}076$; $\Delta\varepsilon = 7{,}09$

Analog werden die folgenden Verbindungen der Formel

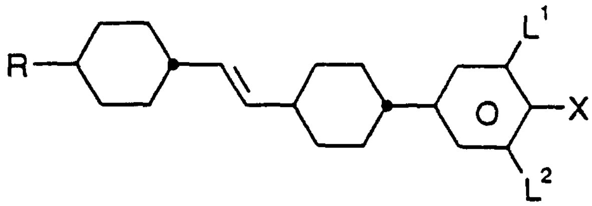

hergestellt:

| R | X | $L^1$ | $L^2$ |
|---|---|---|---|
| $CH_3$-CH=CH | F | F | F |
| $CH_3$-CH=CH-$CH_2$ | F | F | F |
| $CH_3$-$CH_2$-CH=CH | F | F | F |
| $CH_3$-CH=CH-$CH_2$ | F | F | F |
| $CH_3$-$CH_2$-$CH_2$-CH=CH | F | F | F |
| $CH_3$-$CH_2$-CH=CH-$CH_2$ | F | F | F |
| $CH_3$-CH=CH-$CH_2$-$CH_2$ | F | F | F |
| $CH_2$=CH-$CH_2$ | F | F | F |
| $CH_2$=CH-$CH_2$-$CH_2$ | F | F | F |
| $CH_2$=CH | Cl | F | F |
| $CH_3$-CH=CH | Cl | F | F |
| $CH_3$-CH=CH-$CH_2$ | Cl | F | F |
| $CH_3$-$CH_2$-CH=CH | Cl | F | F |
| $CH_3$-CH=CH-$CH_2$ | Cl | F | F |
| $CH_3$-$CH_2$-$CH_2$-CH=CH | Cl | F | F |
| $CH_3$-$CH_2$-CH=CH-$CH_2$ | Cl | F | F |
| $CH_3$-CH=CH-$CH_2$-$CH_2$ | Cl | F | F |
| $CH_2$-CH=$CH_2$ | Cl | F | F |
| $CH_2$=CH-$CH_2$-$CH_2$ | Cl | F | F |
| $CH_2$=CH | $OCF_3$ | F | F |
| $CH_3$-CH=CH | $OCF_3$ | F | F |
| $CH_3$-CH=CH-$CH_2$ | $OCF_3$ | F | F |
| $CH_3$-$CH_2$-CH=CH | $OCF_3$ | F | F |
| $CH_3$-CH=CH-$CH_2$ | $OCF_3$ | F | F |
| $CH_3$-$CH_2$-$CH_2$-CH=CH | $OCF_3$ | F | F |
| $CH_3$-$CH_2$-CH=CH-$CH_2$ | $OCF_3$ | F | F |
| $CH_3$-CH=CH-$CH_2$-$CH_2$ | $OCF_3$ | F | F |
| $CH_2$-CH=$CH_2$ | $OCF_3$ | F | F |
| $CH_2$=CH-$CH_2$-$CH_2$ | $OCF_3$ | F | F |
| $CH_2$=CH | $OCHF_2$ | F | F |
| $CH_3$-CH=CH | $OCHF_2$ | F | F |
| $CH_3$-CH=CH-$CH_2$ | $OCHF_2$ | F | F |
| $CH_3$-$CH_2$-CH=CH | $OCHF_2$ | F | F |
| $CH_3$-CH=CH-$CH_2$ | $OCHF_2$ | F | F |
| $CH_3$-$CH_2$-$CH_2$-CH=CH | $OCHF_2$ | F | F |
| $CH_3$-$CH_2$-CH=CH-$CH_2$ | $OCHF_2$ | F | F |
| $CH_3$-CH=CH-$CH_2$-$CH_2$ | $OCHF_2$ | F | F |
| $CH_2$-CH=$CH_2$ | $OCHF_2$ | F | F |
| $CH_2$=CH-$CH_2$-$CH_2$ | $OCHF_2$ | F | F |

(fortgesetzt)

| R | X | $L^1$ | $L^2$ |
|---|---|---|---|
| $CH_2$=CH | $OCH_2CF_3$ | F | F |
| $CH_3$-CH=CH | $OCH_2CF_3$ | F | F |
| $CH_3$-CH=CH-$CH_2$ | $OCH_2CF_3$ | F | F |
| $CH_3$-$CH_2$-CH=CH | $OCH_2CF_3$ | F | F |
| $CH_3$-CH=CH-$CH_2$ | $OCH_2CF_3$ | F | F |
| $CH_3$-$CH_2$-$CH_2$-CH=CH | $OCH_2CF_3$ | F | F |
| $CH_3$-$CH_2$-CH=CH-$CH_2$ | $OCH_2CF_3$ | F | F |
| $CH_3$-CH=CH-$CH_2$-$CH_2$ | $OCH_2CF_3$ | F | F |
| $CH_2$-CH=$CH_2$ | $OCH_2CF_3$ | F | F |
| $CH_2$=CH-$CH_2$-$CH_2$ | $OCH_2CF_3$ | F | F |
| $CH_2$=CH | $OCHFCF_3$ | F | F |
| $CH_3$-CH=CH | $OCHFCF_3$ | F | F |
| $CH_3$-CH=CH-$CH_2$ | $OCHFCF_3$ | F | F |
| $CH_3$-$CH_2$-CH=CH | $OCHFCF_3$ | F | F |
| $CH_3$-CH=CH-$CH_2$ | $OCHFCF_3$ | F | F |
| $CH_3$-$CH_2$-$CH_2$-CH=CH | $OCHFCF_3$ | F | F |
| $CH_3$-$CH_2$-CH=CH-$CH_2$ | $OCHFCF_3$ | F | F |
| $CH_3$-CH=CH-$CH_2$-$CH_2$ | $OCHFCF_3$ | F | F |
| $CH_2$-CH=$CH_2$ | $OCHFCF_3$ | F | F |
| $CH_2$=CH-$CH_2$-$CH_2$ | $OCHFCF_3$ | F | F |
| $CH_2$=CH | $OCH_2CHF_2$ | F | F |
| $CH_3$-CH=CH | $OCH_2CHF_2$ | F | F |
| $CH_3$-CH=CH-$CH_2$ | $OCH_2CHF_2$ | F | F |
| $CH_3$-$CH_2$-CH=CH | $OCH_2CHF_2$ | F | F |
| $CH_3$-CH=CH-$CH_2$ | $OCH_2CHF_2$ | F | F |
| $CH_3$-$CH_2$-$CH_2$-CH=CH | $OCH_2CHF_2$ | F | F |
| $CH_3$-$CH_2$-CH=CH-$CH_2$ | $OCH_2CHF_2$ | F | F |
| $CH_3$-CH=CH-$CH_2$-$CH_2$ | $OCH_2CHF_2$ | F | F |
| $CH_2$-CH=$CH_2$ | $OCH_2CHF_2$ | F | F |
| $CH_2$=CH-$CH_2$-$CH_2$ | $OCH_2CHF_2$ | F | F |
| $CH_2$=CH | $OC_2F_5$ | F | F |
| $CH_3$-CH=CH | $OC_2F_5$ | F | F |
| $CH_3$-CH=CH-$CH_2$ | $OC_2F_5$ | F | F |
| $CH_3$-$CH_2$-CH=CH | $OC_2F_5$ | F | F |
| $CH_3$-CH=CH-$CH_2$ | $OC_2F_5$ | F | F |
| $CH_3$-$CH_2$-$CH_2$-CH=CH | $OC_2F_5$ | F | F |
| $CH_3$-$CH_2$-CH=CH-$CH_2$ | $OC_2F_5$ | F | F |
| $CH_3$-CH=CH-$CH_2$-$CH_2$ | $OC_2F_5$ | F | F |
| $CH_2$-CH=$CH_2$ | $OC_2F_5$ | F | F |
| $CH_2$=CH-$CH_2$-$CH_2$ | $OC_2F_5$ | F | F |
| $CH_2$=CH | $OC_3F_7$ | F | F |
| $CH_3$-CH=CH | $OC_3F_7$ | F | F |
| $CH_3$-CH=CH-$CH_2$ | $OC_3F_7$ | F | F |
| $CH_3$-$CH_2$-CH=CH | $OC_3F_7$ | F | F |
| $CH_3$-CH=CH-$CH_2$ | $OC_3F_7$ | F | F |
| $CH_3$-$CH_2$-$CH_2$-CH=CH | $OC_3F_7$ | F | F |
| $CH_3$-$CH_2$-CH=CH-$CH_2$ | $OC_3F_7$ | F | F |
| $CH_3$-CH=CH-$CH_2$-$CH_2$ | $OC_3F_7$ | F | F |
| $CH_2$-CH=$CH_2$ | $OC_3F_7$ | F | F |

(fortgesetzt)

| R | X | $L^1$ | $L^2$ |
|---|---|---|---|
| $CH_2$=CH-$CH_2$-$CH_2$ | $OC_3F_7$ | F | F |
| $CH_2$=CH | $OCH_2CHF_2$ | F | F |
| $CH_3$-CH=CH | $OCH_2CHF_2$ | F | F |
| $CH_3$-CH=CH-$CH_2$ | $OCH_2CHF_2$ | F | F |
| $CH_3$-$CH_2$-CH=CH | $OCH_2CHF_2$ | F | F |
| $CH_3$-CH=CH-$CH_2$ | $OCH_2CHF_2$ | F | F |
| $CH_3$-$CH_2$-$CH_2$-CH=CH | $OCH_2CHF_2$ | F | F |
| $CH_3$-$CH_2$-CH=CH-$CH_2$ | $OCH_2CHF_2$ | F | F |
| $CH_3$-CH=CH-$CH_2$-$CH_2$ | $OCH_2CHF_2$ | F | F |
| $CH_2$-CH=$CH_2$ | $OCH_2CHF_2$ | F | F |
| $CH_2$=CH-$CH_2$-$CH_2$ | $OCH_2CHF_2$ | F | F |
| $CH_2$=CH | $OCHFCF_2CF_3$ | F | F |
| $CH_3$-CH=CH | $OCHFCF_2CF_3$ | F | F |
| $CH_3$-CH=CH-$CH_2$ | $OCHFCF_2CF_3$ | F | F |
| $CH_3$-$CH_2$-CH=CH | $OCHFCF_2CF_3$ | F | F |
| $CH_3$-CH=CH-$CH_2$ | $OCHFCF_2CF_3$ | F | F |
| $CH_3$-$CH_2$-$CH_2$-CH=CH | $OCHFCF_2CF_3$ | F | F |
| $CH_3$-$CH_2$-CH=CH-$CH_2$ | $OCHFCF_2CF_3$ | F | F |
| $CH_3$-CH=CH-$CH_2$-$CH_2$ | $OCHFCF_2CF_3$ | F | F |
| $CH_2$-CH=$CH_2$ | $OCHFCF_2CF_3$ | F | F |
| $CH_2$=CH-$CH_2$-$CH_2$ | $OCHFCF_2CF_3$ | F | F |
| $CH_2$=CH | $OCF_2CHFCF_3$ | F | F |
| $CH_3$-CH=CH | $OCF_2CHFCF_3$ | F | F |
| $CH_3$-CH=CH-$CH_2$ | $OCF_2CHFCF_3$ | F | F |
| $CH_3$-$CH_2$-CH=CH | $OCF_2CHFCF_3$ | F | F |
| $CH_3$-CH=CH-$CH_2$ | $OCF_2CHFCF_3$ | F | F |
| $CH_3$-$CH_2$-$CH_2$-CH=CH | $OCF_2CHFCF_3$ | F | F |
| $CH_3$-$CH_2$-CH=CH-$CH_2$ | $OCF_2CHFCF_3$ | F | F |
| $CH_3$-CH=CH-$CH_2$-$CH_2$ | $OCF_2CHFCF_3$ | F | F |
| $CH_2$-CH=$CH_2$ | $OCF_2CHFCF_3$ | F | F |
| $CH_2$=CH-$CH_2$-$CH_2$ | $OCF_2CHFCF_3$ | F | F |
| $CH_2$=CH | $COCF_3$ | F | F |
| $CH_3$-CH=CH | $COCF_3$ | F | F |
| $CH_3$-CH=CH-$CH_2$ | $COCF_3$ | F | F |
| $CH_3$-$CH_2$-CH=CH | $COCF_3$ | F | F |
| $CH_3$-CH=CH-$CH_2$ | $COCF_3$ | F | F |
| $CH_3$-$CH_2$-$CH_2$-CH=CH | $COCF_3$ | F | F |
| $CH_3$-$CH_2$-CH=CH-$CH_2$ | $COCF_3$ | F | F |
| $CH_3$-CH=CH-$CH_2$-$CH_2$ | $COCF_3$ | F | F |
| $CH_2$-CH=$CH_2$ | $COCF_3$ | F | F |
| $CH_2$=CH-$CH_2$-$CH_2$ | $COCF_3$ | F | F |
| $CH_2$=CH | $OCHFCHF_2$ | F | F |
| $CH_3$-CH=CH | $OCHFCHF_2$ | F | F |
| $CH_3$-CH=CH-$CH_2$ | $OCHFCHF_2$ | F | F |
| $CH_3$-$CH_2$-CH=CH | $OCHFCHF_2$ | F | F |
| $CH_3$-CH=CH-$CH_2$ | $OCHFCHF_2$ | F | F |
| $CH_3$-$CH_2$-$CH_2$-CH=CH | $OCHFCHF_2$ | F | F |
| $CH_3$-$CH_2$-CH=CH-$CH_2$ | $OCHFCHF_2$ | F | F |
| $CH_3$-CH=CH-$CH_2$-$CH_2$ | $OCHFCHF_2$ | F | F |

(fortgesetzt)

| R | X | $L^1$ | $L^2$ |
|---|---|---|---|
| $CH_2$-CH=$CH_2$ | $OCHFCHF_2$ | F | F |
| $CH_2$=CH-$CH_2$-$CH_2$ | $OCHFCHF_2$ | F | F |

Mischungsbeispiele

Beispiel 1

| | | | |
|---|---|---|---|
| PCH-5F | 5,0 % | Klärpunkt [°C]: | 92 |
| PCH-7F | 6,0 % | $V_{(10,0,20)}$: | 1,35 V |
| CCP-2OCF$_3$ | 11,0 % | | |
| CCP-3OCF$_3$ | 12,0 % | | |
| CCP-4OCF$_3$ | 10,0 % | | |
| CCP-5OCF$_3$ | 12,0 % | | |
| BCH-3F.F.F | 12,0 % | | |
| BCH-5F.F.F | 11,0 % | | |
| CVCU-V-F | 21,0 % | | |

Beispiel 2

| | | | |
|---|---|---|---|
| PCH-5F | 5,0 % | Klärpunkt [°C]: | 93 |
| PCH-7F | 6,0 % | $V_{(10,0,20)}$: | 1,38 V |
| CCP-2OCF$_3$ | 11,0 % | | |
| CCP-3OCF$_3$ | 12,0 % | | |
| CCP-4OCF$_3$ | 10,0 % | | |
| CCP-5OCF$_3$ | 12,0 % | | |
| BCH-3F.F.F | 12,0 % | | |
| BCH-5F.F.F | 11,0 % | | |
| CVCU-1V-F | 21,0 % | | |

Beispiel 3

| | | | |
|---|---|---|---|
| PCH-5F | 9,0 % | Klärpunkt [°C]: | 96 |
| PCH-6F | 7,2 % | $V_{(10,0,20)}$: | 1,84 V |
| PCH-7F | 5,4 % | | |
| CCP-2OCF$_3$ | 7,2 % | | |
| CCP-3OCF$_3$ | 10,8 % | | |
| CCP-4OCF$_3$ | 8,1 % | | |
| CCP-5OCF$_3$ | 8,1 % | | |
| BCH-3F.F | 10,8 % | | |
| BCH-5F.F | 9,0 % | | |
| ECCP-3OCF$_3$ | 4,5 % | | |
| ECCP-5OCF$_3$ | 4,5 % | | |
| CBC-33F | 1,8 % | | |
| CBC-53F | 1,8 % | | |

(fortgesetzt)

| | | | |
|---|---|---|---|
| CBC-55F | 1,8 % | | |
| CVCU-1V-F | 10,0 % | | |

Beispiel 4

| | | | |
|---|---|---|---|
| PCH-5F | 4,5 % | Klärpunkt [°C]: | 97 |
| PCH-7F | 5,4 % | $V_{(10,0,20)}$: | 1,25 V |
| $CCP-2OCF_3$ | 10,0 % | | |
| $CCP-3OCF_3$ | 11,0 % | | |
| $CCP-4OCF_3$ | 9,0 % | | |
| $CCP-5OCF_3$ | 11,0 % | | |
| BCH-3F.F.F | 11,0 % | | |
| BCH-5F.F.F | 10,0 % | | |
| CCP-3F.F.F | 11,0 % | | |
| CCP-5F.F.F | 8,1 % | | |
| CVCU-V-F | 9,0 % | | |

Beispiel 5

| | | | |
|---|---|---|---|
| PCH-5F | 9,0 % | Klärpunkt [°C]: | 90,6 |
| PCH-6F | 7,2 % | Δn [589 nm, 20 °C]: | 0,0952 |
| PCH-7F | 5,4 % | ν [$mm^2 \cdot s^{-1}$, 20 °C]: | 15 |
| $CCP-2OCF_3$ | 7,2 % | Δε [1 kHz, 20 °C]: | 6,18 |
| $CCP-3OCF_3$ | 10,8 % | | |
| $CCP-4OCF_3$ | 8,1 % | | |
| $CCP-5OCF_3$ | 8,1 % | | |
| BCH-3F.F | 10,8 % | | |
| BCH-5F.F | 9,0 % | | |
| $ECCP-3OCF_3$ | 4,5 % | | |
| $ECCP-5OCF_3$ | 4,5 % | | |
| CBC-33F | 1,8 % | | |
| CBC-53F | 1,8 % | | |
| CBC-55F | 1,8 % | | |
| CVCU-V-F | 10,0 % | | |

Beispiel 6

| | | | |
|---|---|---|---|
| PCH-5F | 3,20 % | Klärpunkt [°C]: | +116 |
| $CCP-2OCF_2$.F.F | 17,04 % | Δε [1 kHz, 20 °C]: | +8,1 |
| $CCP-3OCF_2$.F.F | 16,00 % | | |
| $CCP-5OCF_2$.F.F | 17,04 % | | |
| CUP-2F.F | 5,36 % | | |
| CUP-3F.F | 5,36 % | | |
| CBC-33F | 5,36 % | | |
| CBC-53F | 5,36 % | | |

(fortgesetzt)

| | | | |
|---|---|---|---|
| CBC-55F | 5,28 % | | |
| CVCU-V-F | 20,00 % | | |

Beispiel 7

STN-Mischung bestehend aus

| | | | |
|---|---|---|---|
| PCH-3 | 20,00 % | Klärpunkt [°C]: | 99,5 |
| K6 | 6,40 % | $\Delta\varepsilon$ [1 kHz, 20 °C]: | +10,7 |
| K9 | 7,20 % | $V_{(10,0,20)}$ [V]: | 1,18 |
| CCP-2OCF$_3$ | 4,00 % | | |
| CCP-3OCF$_3$ | 4,00 % | | |
| CCP-4OCF$_3$ | 4,00 % | | |
| CCP-5OCF$_3$ | 4,00 % | | |
| ECCP-2OCF$_3$ | 4,00 % | | |
| ECCP-3OCF$_3$ | 4,00 % | | |
| ECCP-5OCF$_3$ | 4,00 % | | |
| ECCP-3F | 4,00 % | | |
| ECCP-5F | 4,00 % | | |
| CBC-33F | 4,00 % | | |
| CBC-53F | 3,20 % | | |
| CBC-55F | 3,20 % | | |
| CVCU-V-F | 20,00 % | | |

## Patentansprüche

**1.** Flüssigkristallines Medium auf Basis eines Gemisches von polaren Verbindungen mit positiver dielektrischer Anisotropie, **dadurch gekennzeichnet**, daß es ein oder mehrere Vinylenverbindungen der Formel I,

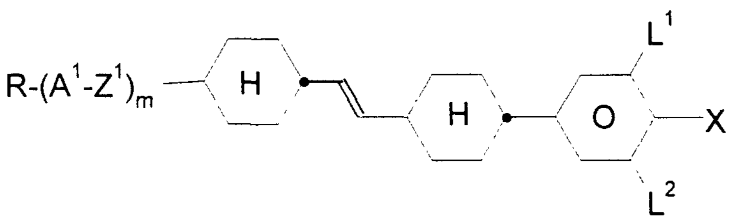

I

worin

R einen unsubstituierten, einen einfach durch CN oder $CF_3$ oder einen mindestens einfach durch Halogen substituierten Alkenylrest mit 2 bis 15 C-Atomen, wobei in diesen Resten auch eine oder mehrere $CH_2$-Gruppen jeweils unabhängig voneinander durch -O-, -S-,

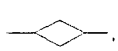

-CO-, -CO-O, -O-CO- oder -O-CO-O- so ersetzt sein können, daß O-Atome nicht direkt miteinander verknüpft sind,

$A^1$ (a) trans-1,4-Cyclohexylenrest, worin auch eine oder mehrere nicht benachbarte $CH_2$-Gruppen durch -O- und/oder -S- ersetzt sein können,

(b) 1,4-Phenylenrest, worin auch eine oder zwei CH-Gruppen durch N ersetzt sein können,

(c) Rest aus der Gruppe 1,4-Cyclohexenylen, 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl, wobei die Reste (a) und (b) ein- oder mehrfach durch CN oder Fluor substituiert sein können,

$Z^1$ -CO-O-, -O-CO-, $-CH_2O-$, $-OCH_2-$, $-CH_2CH_2-$, -CH=CH-, -C≡C- oder eine Einfachbindung, und

X CN, OCN, NCS, Cl, F, halogeniertes Alkyl oder Alkenyl mit 1 bis 7 C-Atomen, worin auch eine oder mehrere $CH_2$-Gruppen durch -O- ersetzt sein können,

$L^1$ und $L^2$ jeweils F, und

m 0 oder 1

bedeuten,
und zusätzlich ein oder mehrere Verbindungen ausgewählt aus der Gruppe bestehend aus den allgemeinen Formeln II bis VI,

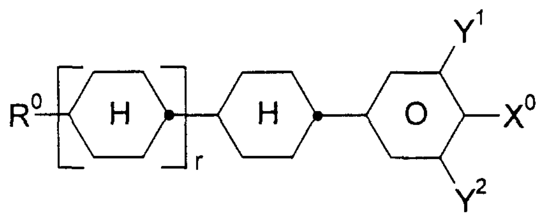

II

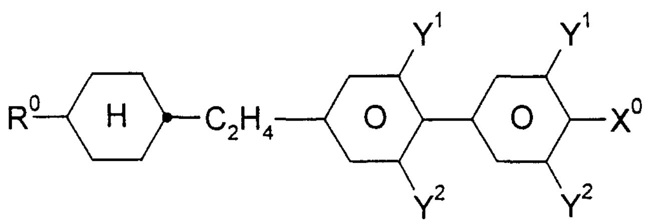

III

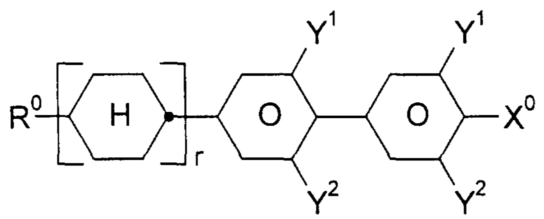

IV

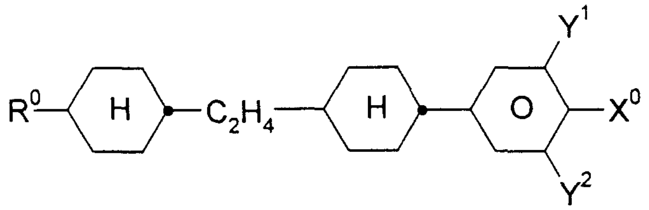

V

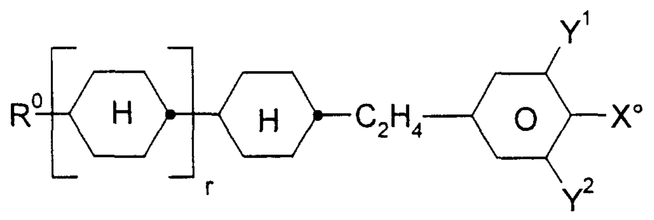

VI

worin

| | |
|---|---|
| $R^0$ | n-Alkyl, Oxaalkyl, Fluoralkyl oder Alkenyl mit jeweils bis zu 9 C-Atomen, |
| $X^0$ | F, Cl, halogeniertes Alkyl, Alkenyl oder Alkoxy mit 1 bis 6 C-Atomen, |
| $Y^1$ und $Y^2$ | jeweils unabhängig voneinander H oder F, und |
| r | 0 oder 1 |

bedeuten,
enthält.

**2.** Flüssigkristallines Medium nach Anspruch 1, **dadurch gekennzeichnet**, daß es ein oder mehrere Verbindungen der Formel I1,

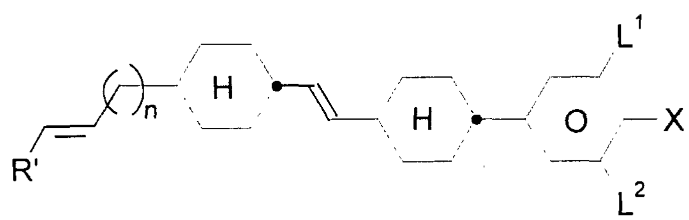

I1

worin

| | |
|---|---|
| R' | H oder n-Alkyl mit 1-5 C-Atomen, |
| n | 0-3, und |

X, $L^1$ und $L^2$ die in Anspruch 1 angegebene Bedeutung haben, bedeutet,
enthält.

**3.** Flüssigkristallines Medium nach Anspruch 1, **dadurch gekennzeichnet**, daß es ein oder mehrere Verbindungen der Formel I enthält, worin R $(CH_2)_n$-CH=CH-R' ist, wobei n 0-3 und R' H oder n-Alkyl mit 1-5 C-Atomen bedeuten.

**4.** Flüssigkristallines Medium nach einem der Ansprüche 1 bis 3, **dadurch gekennzeichnet**, daß es ein oder mehrere Verbindungen der Formel I enthält, worin X CN, F, Cl, $CHF_2$, $OCF_3$, $OCHF_2$, $OCH_2CF_3$, $OC_2F_5$, $OCF_2CHFCF_3$, $OCHFCHF_2$ oder $OCHFCF_3$ bedeutet.

**5.** Flüssigkristallines Medium nach einem der Ansprüche 1 bis 4, **dadurch gekennzeichnet**, daß X Fluor ist.

**6.** Flüssigkristallines Medium nach einem der Ansprüche 1 bis 5, **dadurch gekennzeichnet**, daß es eine Verbindung der Formel 13,

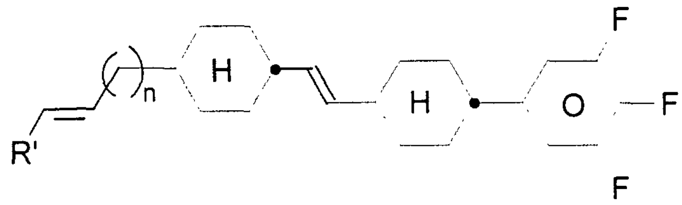 I3

worin

R' H oder Alkyl mit 1-5 C-Atomen, und

n 0-3

bedeuten,
enthält.

**7.** Flüssigkristallines Medium nach einem der Ansprüche 1 bis 6, **dadurch gekennzeichnet**, daß es ein oder mehrere Verbindungen der Formel I enthält, worin R ein geradkettiger Alkenylrest ist und X F, CN, $CHF_2$, $OCH_2CF_3$, $OCF_3$, $OCHF_2$, $OCHFCF_3$, $OC_2F_5$, $OCF_2CHFCF_3$ oder $OCHFCHF_2$ bedeutet.

**8.** Flüssigkristallines Medium nach einem der Ansprüche 1 bis 7, **dadurch gekennzeichnet**, daß der Anteil an Verbindungen der Formel I im Gesamtgemisch 5 bis 50 Gew.% beträgt.

**9.** Flüssigkristallines Medium nach einem der Ansprüche 1 bis 8, **dadurch gekennzeichnet**, daß der Anteil an Verbindungen der Formeln II bis VI im Gesamtgemisch 30 bis 70 Gew.% beträgt.

**10.** Verwendung des flüssigkristallinen Mediums nach Anspruch 1 für elektrooptische Zwecke.

**11.** Elektrooptische Flüssigkristallanzeige enthaltend ein Medium nach Anspruch 1.

## Claims

**1.** Liquid-crystalline medium based on a mixture of polar compounds having positive dielectric anisotropy, **characterized in that** it comprises one or more vinylene compounds of the formula I

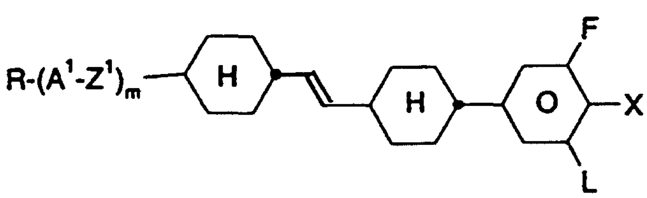 I

in which

R is an alkenyl radical having 2 to 15 carbon atoms which is unsubstituted, monosubtituted by CN or $CF_3$ or at least monosubstituted by halogen, in which radicals one or more $CH_2$ groups may also in each case independently of one another be replaced by -O-, -S-,

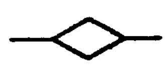,

-CO-, -CO-O, -O-CO- or O-CO-O- in such a way that oxygen atoms are not linked directly to one another,

$A^1$ (a) is a trans-1,4-cyclohexylene radical in which one or more non-adjacent $CH_2$ groups may also

be replaced by -O- and/or -S-,
(b) is a 1,4-phenylene radical in which one or two CH groups may also be replaced by N, or
(c) is a radical from the group consisting of 1,4-cyclohexenylene, 1,4-bicyclo[2.2.2]-octylene, piperidine-1,4-diyl, naphthalene-2,6-diyl, decahydronaphthalene-2,6-diyl and 1,2,3,4-tetrahydronaphthalene-2,6-diyl,

where the radicals (a) and (b) can be substituted one or more times by CN or fluorine,

$Z^1$ is -CO-O-, -O-CO-, $-CH_2O-$, $-OCH_2-$, $-CH_2CH_2-$, -CH=CH-, -C≡C- or a single bond,

X is CN, OCN, NCS, Cl, F, halogenated alkyl or alkenyl having 1 to 7 carbon atoms, in which one or more $CH_2$ groups may also be replaced by -O-,

$L^1$ and $L^2$ are each F, and

m is 0 or 1,

and additionally comprises one or more compounds selected from the group consisting of the general formulae II to VI

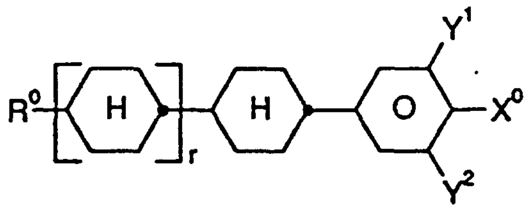

II

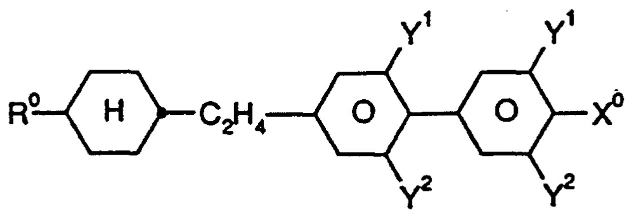

III

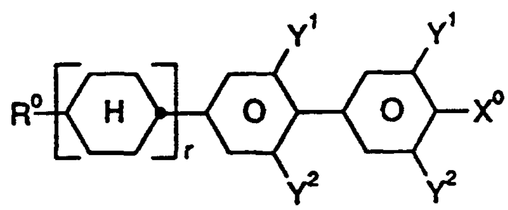

IV

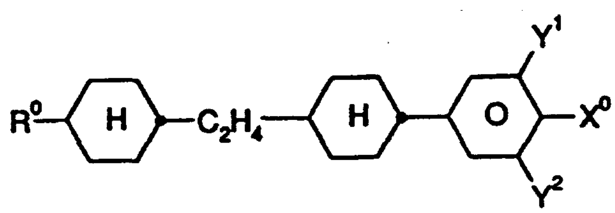

V

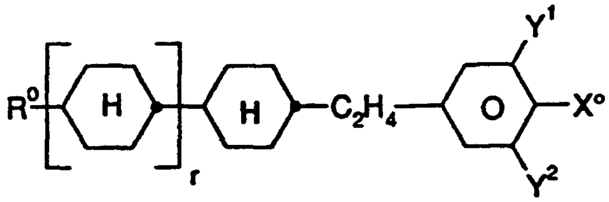

VI

in which

| | |
|---|---|
| $R^0$ | is n-alkyl, oxaalkyl, fluoroalkyl or alkenyl having in each case up to 9 carbon atoms |
| $X^0$ | is F, Cl, halogenated alkyl, alkenyl or alkoxy having 1 to 6 carbon atoms |
| $Y^1$ and $Y^2$ | are each independently H or F, and |
| r | is 0 or 1. |

2. Liquid-crystalline medium according to Claim 1, **characterized in that** it comprises one or more compounds of the formula I1

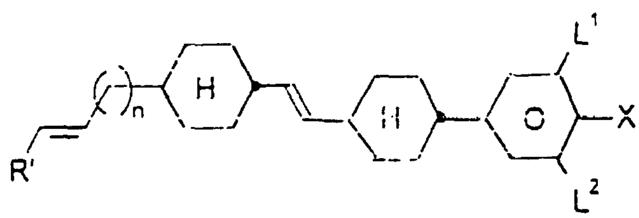

I1

in which

| | |
|---|---|
| R' | is H or n-alkyl having 1 - 5 carbon atoms, |
| n | is 0 - 3, and |

X, $L^1$ and $L^2$ have the meanings given in Claim 1.

3. Liquid-crystalline medium according to Claim 1, **characterized in that** it comprises one or more compounds of the formula I in which R is $(CH_2)_n$-CH=CH-R' in which n is 0-3 and R' is H or n-alkyl having 1 to 5 carbon atoms.

4. Liquid-crystalline medium according to one of Claims 1-3, **characterized in that** it comprises one or more compounds of the formula I, in which X is CN, F, Cl, $CHF_2$, $OCF_3$, $OCHF_2$, $OCH_2CF_3$, $OC_2F_5$, $OCF_2CHFCF_3$, $OCHFCHF_2$ or $OCHFCF_3$.

5. Liquid-crystalline medium according to one of Claims 1 to 4, **characterized in that** X is fluorine.

6. Liquid-crystalline medium according to one of Claims 1 to 5, **characterized in that** it comprises a compound of the formula I3

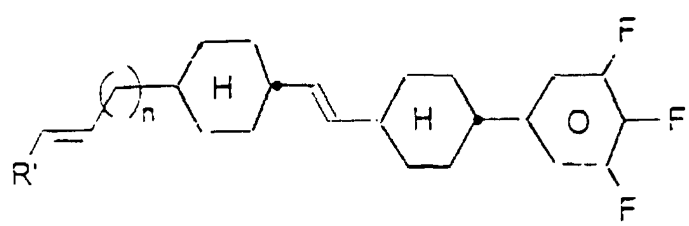

I3

in which

$R^1$ is H or alkyl having 1 - 5 carbon atoms, and
n is 0 - 3.

7. Liquid-crystalline medium according to one of Claims 1 to 6, **characterized in that** it comprises one or more compounds of the formula I in which R is a straight-chain alkenyl radical and X is F, CN, $CHF_2$, $OCH_2CF_3$, $OCF_3$, $OCHF_2$, $OCHFCF_3$, $OC_2F_5$, $OCF_2CHFCF_3$ or $OCHFCHF_2$.

8. Liquid-crystalline medium according to one of Claims 1 to 7, **characterized in that** the proportion of compounds of the formula I in the overall mixture is from 5 to 50% by weight.

9. Liquid-crystalline medium according to one of Claims 1 to 8, **characterized in that** the proportion of compounds of the formulae II to VI in the overall mixture is from 30 to 70% by weight.

10. Use of the liquid-crystalline medium according to Claim 1 for electrooptical purposes.

11. Electrooptical liquid-crystal display containing a medium according to Claim 1.

**Revendications**

1. Milieu cristal liquide à base d'un mélange de composés polaires à anisotropie diélectrique positive, **caractérisé en ce qu'**il contient un ou plusieurs composés vinyles de la formule I,

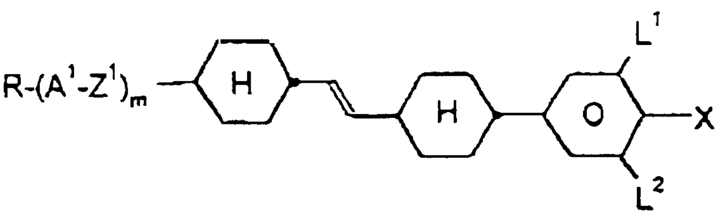

I

R représente un radical alcényle comprenant de 2 à 15 atomes de C, non substitué, substitué simplement par CN ou $CF_3$ ou au moins substitué simplement par un halogène, un ou plusieurs groupes $CH_2$ pouvant être remplacés dans ces radicaux à chaque fois indépendamment les uns des autres par -O-, -S-,

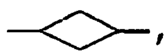

-CO-, -CO-O, -O-CO- ou -O-CO-O- de manière à ce que les atomes de O ne soient pas directement reliés,
$A^1$ représente :

(a) un radical trans-1,4-cyclohexylène, où un ou plusieurs groupes $CH_2$ non voisins peuvent être remplacés par -O- et/ou -S-,

(b) un radical phénylène-1,4, où un ou deux groupes CH peuvent être remplacés par N,

(c) un radical du groupe 1,4-cyclohexenylène, 1,4-bicyclo(2,2,2)-octylène, plpéridine-1,4-diyle, naphtaline-2,6-diyle, décahydronaphtaline-2,6-diyle et 1,2,3,4-tétrahydronaphtaline-2,6-diyle,

les radicaux (a) et (b) pouvant être remplacés une ou plusieurs fois par CN ou du fluor,

$Z^1$ représente -CO-O-, -O-CO-, $-CH_2O-$, $-OCH_2-$, $-CH_2CH_2-$, -CH=CH-, -C≡C- ou une liaison simple, et

X représente CN, OCN, NCS, Cl, F, un alkyle ou un alcényle halogéné comprenant de 1 à 7 atomes de C, où un ou plusieurs groupes $CH_2$ peuvent être remplacés par -O-

$L^1$ et $L^2$ représentent chacun F et

m représente 0 ou 1,

et contient en plus un ou plusieurs composés choisis dans le groupe composé des formules générales II à VI,

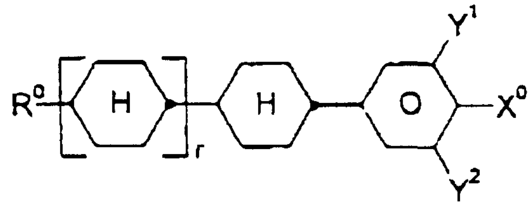

II

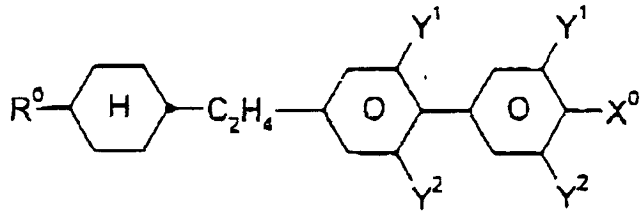

III

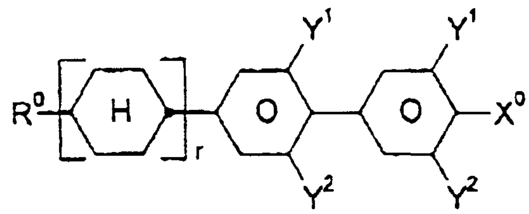

IV

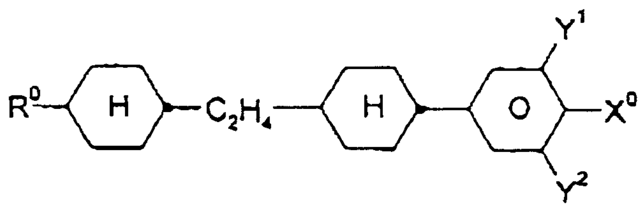

V

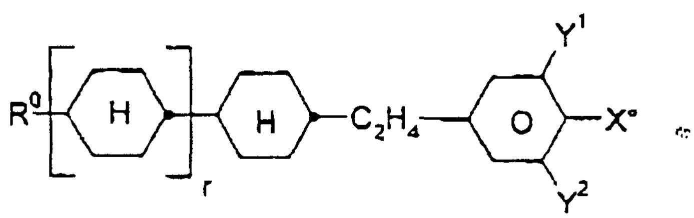

VI

où

$R^0$ représente un n-alkyle, un oxaalkyle, un fluoroalkyle ou un alcényle comprenant à chaque fois jusqu'à 9 atomes de C,

$X^0$ représente F, Cl, un alkyle, alcényle ou alcoxy halogéné comprenant de 1 à 6 atomes de C,

$Y^1$ et $Y^2$ représente chacun indépendamment l'un de l'autre H ou F, et r représente 0 ou 1.

**2.** Milieu cristal liquide selon la revendication 1, **caractérisé en ce qu'**il contient un ou plusieurs composés de formule I1 :

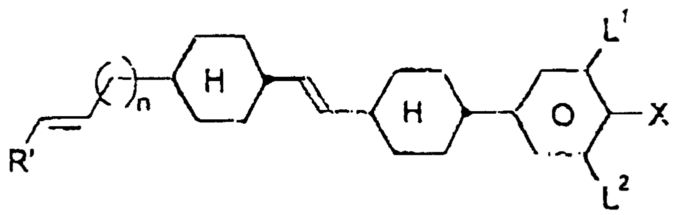

I1

où

R' représente H ou un n-alkyle comprenant de 1 à 5 atomes de C,
N représente 0 - 3, et
X, $L^1$ et $L^2$ ont la signification indiquée à la revendication 1.

**3.** Milieu cristal liquide selon la revendication 1, **caractérisé en ce qu'**il contient un ou plusieurs composés de formule I, où R est égal à $(CH_2)n-CH = CH-R'$, n représentant 0-3 et R' H ou un n-alkyle comprenant de 1 à 5 atomes de C.

**4.** Milieu cristal liquide selon l'une des revendications 1 à 3, **caractérisé en ce qu'**il contient un ou plusieurs composés de la formule 1, où X représente CN, F, Cl, $CHF_2$, $OCF_3$, $OCHF_2$, $OCH_2CF_3$, $OC_2F_5$, $OCF_2CHFCF_3$, $OCHFCHF_2$ ou $OCHFCF_3$.

**5.** Milieu cristal liquide selon l'une des revendications 1 à 4, **caractérisé en ce que** X est du fluor.

**6.** Milieu cristal liquide selon l'une des revendications 1 à 5, **caractérisé en ce qu'**il contient un composé de la formule 13 :

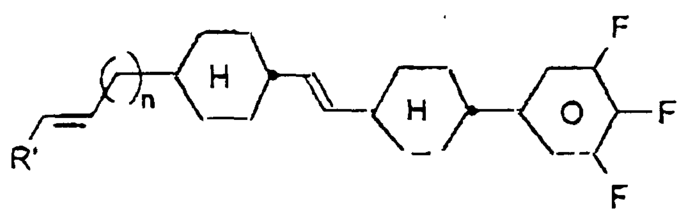

13

où

R' représente H ou un alkyle comprenant de 1 à 5 atomes de C, et
n représente 0 - 3.

**7.** Milieu cristal liquide selon l'une des revendications 1 à 6, **caractérisé en ce qu'**il contient un ou plusieurs composés de la formule I, où R représente un radical alkyle à chaîne linéaire et X représente F, CN, $CHF_2$, $OCH_2CF_3$, $OCF_3$, $OCHF_2$, $OCHFCF_3$, $OC_2F_5$, $OCF_2CHFCF_3$ ou $OCHFCHF_2$.

**8.** Milieu cristal liquide selon l'une des revendications 1 à 7, **caractérisé en ce que** la proportion de composés de la formule I dans le mélange total atteint 5 à 50 % massique.

**9.** Milieu cristal liquide selon l'une des revendications 1 à 8, **caractérisé en ce que** la proportion de composés des formules II à VI dans le mélange total atteint 30 à 70 % en poids.

**10.** Utilisation d'un milieu cristal liquide selon la revendication 1 à des fins électro-optiques.

**11.** Affichages électro-optiques à cristaux liquides contenant un milieu selon la revendication 1.